(19) Europäisches Patentamt
European Patent Office
Office européen des brevets

(11) Numéro de publication : 0 351 332 B1

# (12) FASCICULE DE BREVET EUROPEEN

(45) Date de publication du fascicule du brevet : **24.03.93 Bulletin 93/12**

(21) Numéro de dépôt : **89420247.2**

(22) Date de dépôt : **10.07.89**

(51) Int. Cl.$^5$ : **C07C 317/14,** A01N 41/10, A01N 43/40, A01N 43/04, C07C 317/12, C07C 317/22, C07D 213/61, C07D 213/34, C07C 323/09, C07C 317/10, C07C 317/18

## (54) Composés herbicides et les compositions les contenant.

(30) Priorité : **13.07.88 FR 8809769**

(43) Date de publication de la demande : **17.01.90 Bulletin 90/03**

(45) Mention de la délivrance du brevet : **24.03.93 Bulletin 93/12**

(84) Etats contractants désignés : **AT BE CH DE ES FR GB GR IT LI LU NL SE**

(56) Documents cités : **EP-A- 0 157 712**

(73) Titulaire : **RHONE-POULENC AGROCHIMIE**
**14-20, rue Pierre Baizet**
**F-69009 Lyon (FR)**

(72) Inventeur : **Desbordes, Philippe**
**30 Rue Bancel**
**F-69007 Lyon (FR)**
Inventeur : **Euvrard, Michel**
**33 Route du Mont Thou Saint Romain Au Mont D'Or**
**F-69270 Fontaines Sur Saone (FR)**

(74) Mandataire : **Brachotte, Charles et al**
**RHONE-POULENC AGROCHIMIE DPI BP 9163**
**F-69263 Lyon Cedex 09 (FR)**

EP 0 351 332 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention est relative à de nouveaux composés, à leur utilisation à titre d'herbicides notamment sous forme de composition herbicide, à un procédé de contrôle des mauvaises herbes à l'aide de ces composés ou de ces compositions.

Un objet de la présente invention est donc de proposer des composés utiles en pré ou post émergence comme herbicides.

Un autre objet de la présente invention est de proposer des composés utiles en pré ou post émergence comme herbicides antigraminées.

Un autre objet de la présente invention est de proposer des composés utiles en pré ou post émergence comme herbicides sélectifs du maïs et de nombreuses cultures dicotylédones (notamment soja, colza, tournesol, coton) et autres monocotyledones (blé, riz).

Définition générale de l'invention

Composés de formule :

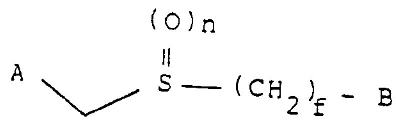

I

dans laquelle :
n = 0, 1, 2
f = 0, 1
A est choisi parmi les groupes

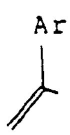
($A_1$)

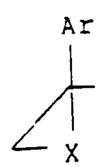
($A_2$)

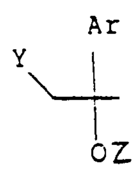
($A_3$)

dans lesquels :
Ar est choisi parmi les groupes

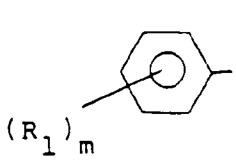
Ar-1

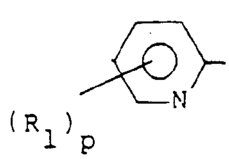
Ar-2

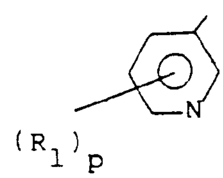
Ar-3

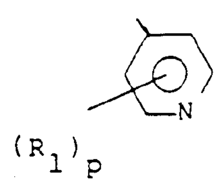
Ar-4

X étant un atome d'oxygène ou de soufre,
$R_1$ étant un atome d'halogène (notamment Cl ou Br ou F), un groupe $C_1$-$C_4$ alkyle, $C_1$-$C_4$ alkoxy, $C_1$-$C_4$ haloalkyle, $C_1$-$C_4$ haloalkoxy, nitro, cyano, $C_6$-$C_{10}$ aryle (notamment phényle ou naphtyle), $C_7$-$C_{11}$ aralkyle (notamment benzyle), $C_6$-$C_{10}$ aryloxy (notamment phénoxy ou napthoxy) éventuellement substitué par 1 ou 2 atomes d'halogène, $C_7$-$C_{11}$ aralkyloxy (notamment benzyloxy) éventuellement substitué par 1 ou 2 atomes d'halogène,
m = 0, 1, 2, 3, 4, 5
p = 0, 1, 2, 3, 4
les différents radicaux $R_1$ étant identiques ou différents lorsque m ou p est supérieur ou égal à 2.
Y est un atome de chlore ou de brome ou d'iode ou un groupement $OSO_2R_2$,

$R_2$ étant un groupement $C_1$-$C_6$ alkyle, $C_6$-$C_{10}$ aryle, $C_7$-$C_{11}$ aralkyle, les dits groupements étant éventuellement substitués par 1 à 3 halogènes ou groupements $C_1$-$C_4$ alkyle, $C_1$-$C_4$ alkoxy, $C_1$-$C_4$ haloalkyle, $C_1$-$C_4$ haloalkoxy, nitro,

Z étant un atome d'hydrogène ou un groupe (C=O)$R_{11}$,

$R_{11}$ est hydrogène, $C_1$-$C_4$ alkyle, $C_1$-$C_4$ haloalkyle,

B est choisi parmi les groupes $C_1$-$C_{10}$ alkyle, $C_3$-$C_{10}$ cycloalkyle, ces groupes étant éventuellement substitués par 1 à 6 atomes d'halogène ou choisi parmi les groupes

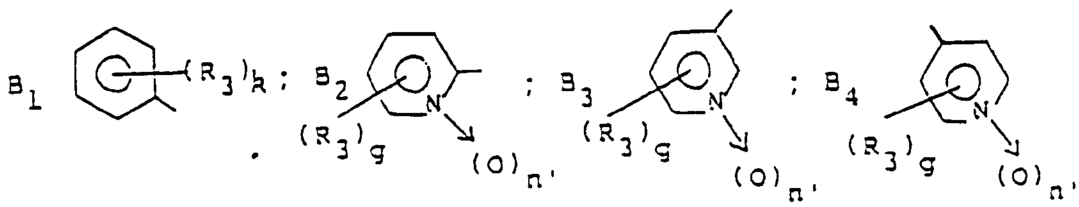

$B_1$ ; $B_2$ ; $B_3$ ; $B_4$

$R_3$ ayant l'une des significations indiquées pour $R_1$ ou $NR_4R_5$, $S(O)_hR_5$, (C=O)$R_7$,

$R_4$, $R_5$ identiques ou différents sont hydrogène,

$C_1$-$C_4$ alkyle, ou $C_6$-$C_{10}$ aryle,

$R_5$ est $C_1$-$C_4$ alkyle,

$R_7$ est $C_1$-$C_4$ alkyle, est $C_1$-$C_4$ alkoxy,

$C_1$-$C_4$ haloalkoxy, $NR_9R_{10}$,

$R_9$, $R_{10}$ identiques ou différents sont hydrogène ou $C_1$-$C_4$ alkyle,

k = 0, 1, 2, 3, 4, 5,

g = 0, 1, 2, 3, 4

h = 0, 1, 2,

n' = 0, 1.

Comme art antérieur décrivant des composés ayant une structure comprenant à la fois deux noyaux aromatiques et un atome de soufre, ce dernier étant éventuellement sous forme de groupe S(O) ou $S(O)_2$, on citera :

-EP-A- 0.157.712 qui décrit essentiellement des (arylthio)pyridylalkanols de formule :

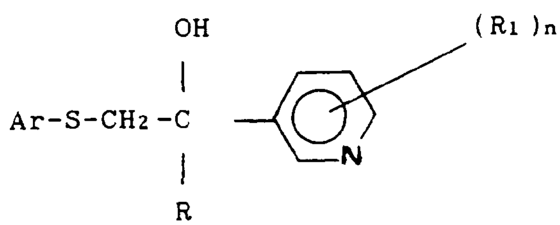

et leur emploi comme agent fongicide ;

-EP-A-0.281.110 et EP-A-0.326.170 qui décrivent essentiellement des arylsulfonamides di N-substitués dont l'un des substituants comprend un autre noyau aromatique et leur emploi comme agent herbicide ; à noter que ces documents ont tous deux été déposés antérieurement à la date de dépôt de la présente invention, mais publiés postérieurement à cette date de dépôt.

<u>Variantes préférées</u>

Selon des modes de réalisation préférés on choisira les variantes suivantes prises ou non en combinaison :

- n = 2
- X = 0
- Z = H
- m inférieur ou égal à 2
- p inférieur ou égal à 2
- k inférieur ou égal à 2
- g inférieur ou égal à 1
- $R_1$ est halogène, nitro, trifluorométhyle, méthoxy, méthyle.

Les composés de formule I et les composés éventuellement utilisables à titre d'intermédiaires dans les procédés de préparation et qui seront définis à l'occasion de la description de ces procédés peuvent exister sous une ou plusieurs formes d'isomères selon le nombre de centres asymétriques de la molécule. L'invention concerne donc aussi bien tous les isomères optiques que leurs mélanges racémiques et les diastéréoisomères correspondants. La séparation des diastéréoisomères et/ou des isomères optiques peut s'effectuer selon les méthodes connues en soi.

Procédés de préparation

Méthode A

Les composés de formule (I) pour lesquels n = 2, et A est (Al), les autres substituants ayant la même définition que celle indiquée dans la définition générale de l'invention peuvent être obtenus par mise en contact d'un composé de formule :

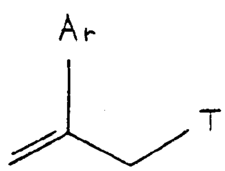

II

dans laquelle Ar, $R_1$ et m ou p ont la même signification que dans la définition générale de l'invention,
T est un atome de chlore ou de brome, avec un composé de formule :

$$MSO_2\ (CH_2)_f - B \qquad III$$

f, B ayant la même définition que celle indiquée dans la définition de l'invention,
M étant un atome de métal alcalin ou alcalino-terreux (notamment Li, K, Na).

La réaction est généralement effectuée dans un solvant aprotique dipolaire notamment le diméthylformamide, la N-méthyl pyrrolidone, à une température comprise entre 25°C et 150°C (de préférence 60°C à 120°C) et dans un rapport molaire II:III compris entre 1 et 10 (de préférence 1 et 2).

Cette réaction est notamment connue par "J. March, Advanced Organic Chemistry" Ed. Mc Graw-Hill (1985) p. 363.

Les composés de formule (II) où T est chlore, et Ar est Ar-1 (noyau phényle) sont préparés par chloration d'un composé 2-phényl 1-propène de formule :

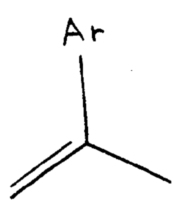

IV

dans laquelle Ar est Ar-1 (noyau phényle),
$R_1$ et m ayant la même signification que dans la définition de la formule générale, au moyen du réactif $Ca(OCl)_2/CO_2$.

Cette réaction est décrite par S.G. Hegde et J. Wolinsky Tetrahedron Letters (1981), 22, 5019.

Les composés de formule (II) où T est chlore, Ar ayant la même définition que celle indiquée dans la définition générale de l'invention, peuvent également être préparés par chloration des composés de formule (IV) sus indiqués au moyen de N-chloro succinimide en présence de bisaryldisélénide suivant le procédé de K.B. Sharpless et T. Hori J. Org. Chem. (1979), 44, 4204.

Les composés de formule (II) où T est chlore ou brome, Ar ayant la même définition que celle indiquée dans la définition générale de l'invention, peuvent également être préparés par halogénation radicalaire thermique ou photochimique par la N-halogéno succinimide du composé de formule (IV), dans un solvant aprotique comme le tétrachlorure de carbone ou en l'absence de solvant avec ou non un initiateur de radicaux libres à une température de 20°C à 170°C (de préférence 80°C à 100°C) suivant S.F. Reed J. Org. Chem. (1965), 30, 3258. Ils peuvent encore être préparés par halogénation des composés de formule (II) où T est OH, Ar ayant la même définition que celle indiquée dans la définition générale de l'invention, avec un agent d'halogénation

tel que $SOCl_2$, $POCl_3$, $PBr_3$, J. March ibid. p. 382-384 ou avec le réactif LiCl / $CH_3SO_2Cl$ / collidine suivant E.W. Collington et A.I. Meyers J. Org. Chem. (1971), 36, 3044.

Les composés de formule (II) où T est OH, Ar ayant la même définition que celle indiquée dans la définition générale de l'invention, peuvent être préparés par oxydation allylique du composé de formule (IV) par l'oxyde de sélénium catalytique ou non en présence d'un oxydant comme l'hydroperoxyde de tertiobutyle dans un solvant inerte comme les solvants halogénés (de préférence $CH_2Cl_2$) ou le tertiobutanol, en présence d'acide minéral ou organique selon M.A. Umbreit et K.B. Sharpless J. Amer. Chem. Soc. (1977), 99, 5526.

Les composés de formule (IV) peuvent être obtenus par déshydratation d'un composé 2-aryl 2-propanol de formule :

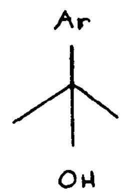

V

dans laquelle Ar, $R_1$ et m ou p ont la même signification que dans la définition générale de l'invention, par des agents de déshydratation comme $P_2O_5$, $KHSO_4$, $POCl_3$ / pyridine J. March ibid. p. 901-903.

Les composés de formule (V) peuvent être préparés par mise en contact de l'acétophénone ou acétylpyridine ou dérivé d'acide de formule :

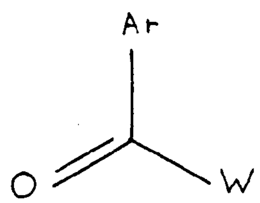

VI

dans laquelle Ar, $R_1$ et m ou p ont la même signification que dans la définition générale de l'invention et W est méthyle, alkoxy (ester benzoïque correspondant) ou chlore avec un ou deux équivalents d'halogènure de méthylmagnésium suivant J. March ibid. p. 816-822.

Les composés de formule (VI) sont obtenus de manière connue en soi.

Les composés de formule (III) peuvent être préparés par réduction des halogénures de sulfonyle correspondants (généralement chlorure) par le zinc, l'iodure de sodium ou de potassium, le sulfite de sodium selon J. March ibid. p. 445-446. Les halogénures de sulfonyle peuvent être préparés selon J. March ibid. p. 1172.

Les composés de formule (III) peuvent être également préparés par action d'un organométallique (habituellement lithien) de formule :

$$M\,(CH_2)_f\text{-}B \qquad \text{IIIA}$$

f, B ayant la même signification que celle indiquée dans la définition de l'invention, M étant notamment lithium, avec l'anhydride sulfureux $SO_2$, à une température comprise entre -78°C et 20°C (de préférence -78°C à -40°C) en l'absence ou en présence d'un solvant aprotique comme l'éther éthylique ou le tétrahydrofuranne selon H.W. Pinnick et M.A. Reynolds J. Org. Chem. (1979), 44, 160, et J. March ibid. p. 550.

Les composés de formule (IIIA) sont obtenus de manière connue en soi.

Les composés de formule (III) dans laquelle B est $B_1$, $B_2$, $B_3$, $B_4$ et f = O peuvent être également préparés par action du sel de diazonium d'aryle correspondant avec l'anhydride sulfureux $SO_2$ en présence de cuivre selon W.E. Truce et A.H. Murphy Chem. Rev. (1951), 48, 69 et ref. citées.

Méthode B

Les composés de formule (I) dans laquelle A est ($A_1$) et n = 0, 1 ou 2 peuvent être préparés par action du sel alcalin d'un thiolate d'aryle ou d'alkyle de formule :

$$M' - S - (CH_2)_f\,B \qquad \text{VII}$$

dans laquelle M' est un atome de métal alcalin ou alcalinoterreux notamment Na ou K, f et B ayant la même définition que celle indiquée dans la définition de l'invention sur un composé de formule (II) où T est halogène

précédemment décrit dans un solvant inerte protique ou aprotique tel que les cétones, les alcools, le tétrahydrofuranne, l'acétonitrile, à une température de 0°C à 80°C (généralement 25°C à 60°C) dans un rapport molaire II:VII compris généralement entre 1 et 10 (de préférence 1 et 2).

Le sulfure ainsi obtenu (n=0) peut être oxydé en sulfoxyde (n=1) par un équivalent d'oxydant à une température de - 70°C à 5°C (généralement 0°C) ou être oxydé en sulfone (n=2) par deux ou plus équivalents d'oxydant à une température de 0°C à 60°C (généralement 10°C à 30°C) par de nombreux oxydants comme $KMnO_4$, $H_2O_2$, $CH_3CO_3H$, acides perbenzoïques, $KHSO_5$ et d'autres suivant de très nombreuses méthodes connues (J. March ibid. p. 1089-1090).

Dans le cas où B est $B_2$, $B_3$, $B_4$ et n'=0, on choisira un oxydant spécifique du soufre comme l'hydrogénopersulfate de tétrabutylammonium selon B.M. Trost et R. Braslau J. Org. Chem. (1988), 53, 532.

Méthode C

Dans le cas où n = 2 et A est ($A_1$) un autre procédé de préparation des composés de formule (I) consiste à faire réagir le composé de formule (IV), précédemment décrit, sur le composé de formule (III) précédemment décrit, dans un solvant inerte aprotique comme l'acétone, l'acétate d'éthyle, l'acétonitrile en présence d'un solvant protique (de préférence $H_2O$) avec un équivalent d'iode à température ambiante selon L.M. Harwood et M. Julia Tetrahedron (1980), 36, 483 et T. Kobayaski et al. Chem. Letters (1987), 1209.

Méthode D

Les composés de formule (I) dans laquelle A est ($A_1$), n = 1, 2, les autres substituants ayant la même définition que celle indiquée dans la définition générale de l'invention peuvent être obtenus par mise en contact de l'anion d'un aryl ou alkylsulfonométhyl phosphonate de dialkyle ou aryle de formule :

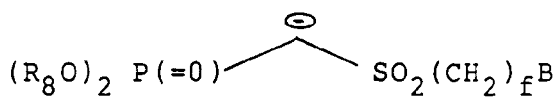

VIII

ou de l'anion d'un aryl ou alkylsulfinométhyl phosphonate de dialkyle ou aryle de formule :

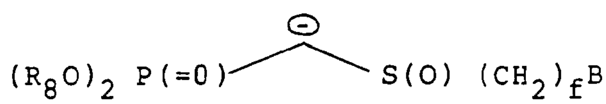

IX

formules dans lesquelles $R_8$ est un groupement $C_1$-$C_4$ alkyle ou $C_6$-$C_{10}$ aryle, f et B ayant la même définition que celle indiquée dans la définition générale de l'invention, sur une acétylpyridine ou acétophénone de formule (VI) dans laquelle W est méthyle et Ar, $R_1$ et m ou p ont la même signification que dans la définition générale de l'invention.

La réaction est effectuée dans un rapport molaire VI : VIII ou IX compris généralement entre 1 et 5 (de préférence 1 et 2), dans un solvant aprotique comme le tétrahydrofuranne en présence ou non de solvant dipolaire aprotique comme l'hexaméthylphosphotriamide, à une température de - 78°C à 80°C (de préférence - 78°C à 40°C) pour fournir les composés de formule X :

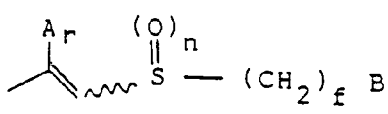

X

dans laquelle Ar, $R_1$, m, p, f et B ont la même signification que dans la définition générale de l'invention et n = 1 ou 2, selon H. Fillion et al. J. Heterocyclic Chem. (1978), 15, 753.

Les anions de formules (VIII) et (IX) sont préparés par action d'une base comme NaH, Buli sur les composés correspondants.

Les composés de formule (I) dans laquelle A est ($A_1$) et n = 1 ou 2, sont obtenus par isomérisation en milieu basique des composés de formule (X) par 1 à 5 équivalents de base (de préférence 2) comme le tertiobutylate de potassium, le 1,8-diazabicyclo [5,4,0] undéc-7-ène dans des solvants comme l'acétonitrile, le diméthylsulfoxyde, le terbutanol à une température de 25°C à 100°C (de préférence 25°C à 60°C) selon D.E. O'Connors et W.I. Lyness J. Amer.Chem. Soc. (1964), 86, 3044 ou T. Kobayashi et al. Chem. Letters (1987), 1209, les aryl (ou alkyl)sulfonométhylphosphonate de dialkyle (ou aryle) de formule (VIII) ou les aryl (ou alkyl)sulfinométhylphosphonate de dialkyle (ou aryle) de formule (IX) sont préparés par oxydation des aryl (ou alkyl)thiométhylphosphonate de dialkyle (ou aryle) suivant les méthodes décrites dans la méthode B.

Les aryl (ou alkyl)thiométhylphosphonate de dialkyle (ou aryle) sont préparés par une réaction d'Arbuzov des chlororméthylaryl (ou alkyl) sulfures sur le trialkyle (ou aryle) phosphite correspondant (J. March ibid. p. 848) suivant des méthodes bien connues.

Les chlorométhylaryl (ou alkyl) sulfures sont préparés par chlorométhylation des thiols correspondants suivant des méthodes connues.

Méthode E

Les composés de formule (I) dans laquelle A est ($A_1$), et n = 2, les autres substituants ayant la même définition que celle indiquée dans la définition générale de l'invention peuvent être obtenus dans une première étape, par mise en contact de l'anion ou dianion d'une méthylaryl ou alkylsulfone de formule :

$$CH_3\,SO_2\,(CH_2)_f\text{-}B \qquad \text{IIIB}$$

f, B ayant la même définition que celle indiquée dans la définition générale de l'invention, sur une acétophénone ou une acétylpyridine de formule (VI) dans laquelle W est méthyle et Ar, $R_1$ et m ou p ont la même définition que dans la définition générale de l'invention pour fournir les composés de formule :

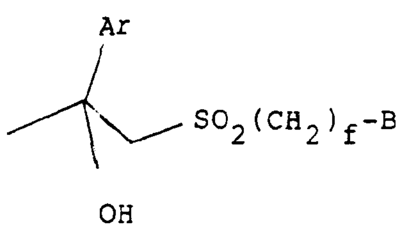

XI

dans laquelle Ar, $R_1$, m ou p, f et B ont la même signification que dans la définition générale de l'invention.

La réaction est effectuée dans un rapport molaire VI : IIIB compris généralement entre 1 et 5 (de préférence 1 et 2), dans un solvant aprotique comme le tétrahydrofuranne à une température de -70°C à 50°C (de préférence -70°C à 20°C) selon N. Hanack et K. Laping, Tetrahedron Letters (1977), 4493 ou D.F. Tavares et P.F. Vogt Can. J. Chem. (1967), 45, 1519.

Les anions des composés de formule (IIIB) sont préparés par action d'un ou plus équivalents d'organométallique comme le butyllithium ou les halogénures d'alkylmagnésium sur les composés correspondants.

Les composés de formule (I) dans laquelle A est ($A_1$) et n = 2 sont obtenus dans une deuxième étape, par déshydratation des composés de formule (XI) suivant la méthode décrite dans la méthode A pour la déshydratation des composés de formule (V).

La déshydratation fournit un mélange de composés de formule (I) dans laquelle A est ($A_1$) et n = 2, et de composés de formule (X) qui peut être isomérisé en milieu basique suivant la méthode décrite dans la méthode D pour donner les composés de formule (I) dans laquelle A est ($A_1$) et n = 2.

Les méthylaryl ou alkylsulfones de formules (IIIB) sont préparées par action d'un sulfinate d'aryle ou d'alkyle de formule (III) sur l'iodure de méthyle ou par oxydation des méthylaryle ou alkylsulfures correspondants préparés par action d'un thiolate d'aryle ou d'alkyle de formule (VIII) sur l'iodure de méthyle suivant les méthodes décrites dans les méthodes A et B.

Les méthylarylsulfones de formule (IIIB) peuvent également être préparés par méthylsulfonylation directe du noyau aromatique correspondant, par le chlorure de méthylsulfonate en présence d'acide méthanesulfonique et d'acide trifluorométhanesulfonique selon M. Ono et al. Chem. Letters (1988), 395.

Méthode F

Les composés de formule (I) dans laquelle A est ($A_3$), Y est Cl ou Br , Z est H, les autres substituants ayant la même définition que celle indiquée dans la définition générale de l'invention peuvent être obtenus par halogénation en milieu aqueux des composés précédemment obtenus de formule (I) où A est ($A_1$). Les agents d'halogénation sont la N-halogéno succinimide, le N-halogéno acétamide dans un rapport molaire I : agent d'halogénation de 1 à 10 (de préférence 2 à 4) dans un mélange de proportion 5/95 à 90/10 (de préférence

5/95 à 20/80) d'eau et d'un solvant soluble dans l'eau comme le diméthylsulfoxyde, le dioxanne, l'acétone et autres à une température de 0°C à 60°C (de préférence 20°C à 40°C) en présence ou non d'un tampon qui peut être par exemple l'acide acétique / acétate de sodium selon H.O. House J. Amer. Chem. Soc. (1955), 77, 3070 ou J. March ibid. p. 726-727.

Méthode G

Les composés de formule (I) dans laquelle A est ($A_3$), Y est Cl, Br, I, Z est H, peuvent être préparés par substitution nucléophile des composés de formule (I) dans laquelle A est ($A_3$), Y est $OSO_2R_2$, Cl, Br, Z est H, par le sel alcalin (sodium, potassium, lithium) d'halogénure (Y = Cl, Br, I) en rapport molaire I : sel de 1 à 10 (de préférence 1 à 3) dans des solvants comme l'acétone, la méthyléthylcétone, le glyme, le diglyme, le diméthylformamide, à une température de 25°C à 180°C (de préférence 40°C à 160°C) selon J. March ibid. p. 381-382.

Méthode H

Les composés de formule (I) dans laquelle A est ($A_3$), Y est Cl ou Br, Z est H et n = 2, les autres substituants ayant la même définition que celle indiquée dans la définition générale de l'invention, peuvent être préparés par mise en contact de l'anion d'une méthylaryl ou alkylsulfone de formule (IIIB), f et B ayant la même définition que celle indiquée dans la définition générale de l'invention, sur une ω -halogénoacétophénone ou halogénoacétylpyridine de formule :

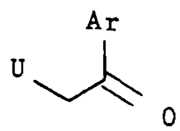

XII

dans laquelle U est un atome de chlore ou de brome, Ar, $R_1$, m ou p ayant la même définition que celle indiquée dans la définition générale de l'invention, dans un rapport molaire XII : IIIB compris généralement entre 1 et 5 (de préférence 1 et 2), dans un solvant aprotique comme le tétrahydrofuranne, le diméthoxyéthane à une température de -100°C à 0°C (de préférence -70°C à -20°C) selon les références décrites dans la méthode E.

Les anions des composés de formule (IIIB) et les composés de formule (IIIB) sont préparés suivant les méthodes décrites dans la méthode E.

Les composés de formules (XII) sont préparés suivant des méthodes en soi connues.

Méthode I

Les composés de formule (I) dans laquelle A est ($A_3$), Y est Br ou Cl, Z est H, les autres substituants ayant la même définition que celle indiquée dans la définition générale de l'invention, peuvent être préparés par ouverture des composés de formule (I) dans laquelle A est ($A_2$), X est O, les autres substituants ayant la même définition que celle indiquée dans la définition générale de l'invention, par action d'un réactif donneur d'halogénure $Y^-$, Y étant l'atome de chlore ou de brome, comme les acides halohydriques HY, l'halogènure de magnésium $MgY_2$, les complexes $NiLi_2Y_4$ selon R.D. Dawe, T.F. Molinski et J.V. Turner Tetrahedron Letters (1984), 25, 2061 ou J.March ibid., p. 385-386.

Méthode J

Les composés de formule (I) dans laquelle A est ($A_3$), Y est Br ou Cl, Z est ester, les autres substituants ayant la même définition que celle indiquée dans la définition générale de l'invention, peuvent être préparés par halogénation par la N-halogéno succinimide, le N-halogéno acétamide, des composés précédemment obtenus de formule (I) où A est ($A_1$) en présence d'un acide $R_{11}CO_2H$, $R_{11}$ ayant la même définition que celle indiquée dans la définition générale de l'invention, dans un rapport molaire I : agent d'halogénation de 1 à 10 (de préférence 1 à 3), dans un mélange de proportion 100/0 à 10/90 (de préférence 100/0 à 50/50) d'acide et d'un solvant comme l'acétone, le dioxanne et autres à une température de 0°C à 60°C (de préférence 0°C à 20°C) en présence ou non du sel de l'acide $R_{11}CO_2M$, M étant l'atome de sodium, potassium ou lithium dans un rapport molaire I : sel d'acide de 1 à 10 (de préférence 5) selon V.L. Heasley et R.A. Skidgel J. Org. Chem. (1974), 89, 3953.

Les composés de formule (I) dans laquelle A est ($A_3$), Y est I, Z est ester, peuvent être préparés par substitution nucléophile des composés de formule (I) où A est ($A_3$), Y est Br, Z est ester suivant la méthode décrite dans la méthode G.

Méthode K

Les composés de formule (I) dans laquelle A est ($A_3$), Y est $OSO_2R_2$, Z est H, les autres substituants ayant la même définition que celle indiquée dans la définition générale de l'invention peuvent être obtenus par action de l'halogénure $R_2SO_2$-Hal (Hal étant de préférence l'atome de chlore) sur le composé diol de formule :

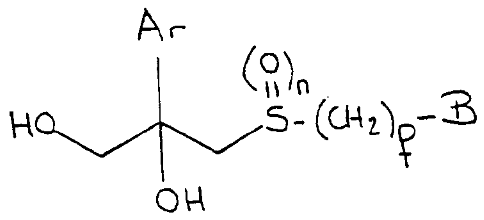

XIII

en présence d'une base azotée comme la pyridine, la triéthylamine dans un rapport molaire XIII : halogénure : base comprise entre 1:1:1 et 1:5:100 (de préférence 1:1:1 et 1:2:5) dans un solvant comme l'éther, le diméthoxyéthane, le tétrahydrofuranne et autres selon J. March ibid. p. 357-358.

Le composé de formule (XIII) peut être préparé par perhydroxylation des composés de formule (I) où A est ($A_1$) (n = 1, 2) suivant des méthodes connues (J. March ibid. p. 732-734) comme par exemple l'action catalytique du tétraoxyde d'osmium en présence d'un oxydant comme l'hydroperoxyde de tertiobutyle dans un solvant inerte comme l'acétone ou autre en présence ou non d'un sel d'ammonium selon Akaski, R.E. Palermo et K.B. Sharpless J. Org. Chem (1978), 43, 2063.

Méthode L

Les composés de formule (I) dans laquelle A est ($A_2$), X est 0, les autres substituants ayant la même signification que celle indiquée dans la définition générale de l'invention peuvent être obtenus par cyclisation d'un composé de formule (I) où A est ($A_3$) et Y est Cl, Br, I, Z est H, en présence de sel d'argent $Ag_2O$ dans un rapport molaire I : $Ag_2O$ compris entre 1 et 5 (de préférence 2 et 3) dans un solvant aprotique comme l'éther, le tétrahydrofuranne, le diméthoxyéthane à une température de 25°C à 150°C (de préférence 60°C à 85°C) suivant J.D. Mc Clure J. Org. Chem. (1967), 32, 3888.

Dans le cas où n = 0 ou 1 ces mêmes composés de formule (I) dans laquelle A est ($A_2$) peuvent également être préparés par cyclisation en milieu basique (NaOH, KOH, $K_2CO_3$ et autres) des composés de formule (I) où A est ($A_3$), Y est Cl, Br, I, $OSO_2R_2$, Z est H, selon J. March ibid. p. 343.

Les composés de formule (I) où A est ($A_2$) et n = 2 ou 1 peuvent être préparés par oxydation des composés de formule (I) où A est ($A_2$) et n = 1 ou 0 suivant les méthodes décrites dans la méthode B.

Méthode M

Les composés de formule (I) dans laquelle A est ($A_2$), n = 2, X = 0, les autres substituants ayant la même signification que celle indiquées dans la définition générale de l'invention peuvent être obtenus par oxydation d'un composé de formule (I) où A est ($A_1$), n = 2, par des agents d'oxydation comme les peracides organiques dans un rapport molaire I : oxydant compris entre 1 et 10 (de préférence 1 à 3) en milieu tamponné par le sel alcalin ou alcalino-terreux d'un acide organique selon J. March ibid. p. 735-736.

Méthode N

Les composés de formule (I) où A est ($A_2$) et X = O, les autres substituants ayant la même signification que celle indiquées dans la définition générale de l'invention, peuvent être préparés par déshydratation des composés de formule (XIII), par des agents de déshydratation comme le diméthylacétaldiméthylformamide, le réactif diéthylazodicarboxylate/triphénylphosphine selon C. Cortez et R.G. Harvey, Org. Synth. (1978), 58, 12, ou J.T. Carlock et M.P. Mack Tetrahedron Letters (1978), 5153.

Méthode O

Les composés de formule (I) où A est ($A_2$) et X est S, les autres substituants ayant la même définition que celle indiquée dans la définition générale de l'invention, sont préparés par réaction des composés de formule (I) où A est ($A_2$) et X est 0 avec un sel alcalin (généralement potassium) du thiocyanate de 1 à 10 équivalents (de préférence 1 à 3) dans des solvants comme l'éthanol, l'acétonitrile et autres à des températures de 25°C à 120°C (de préférence 25°C à 60°C) selon Org. Synth., IV, 232.

Ces mêmes composés peuvent être également préparés d'après de nombreuses autres méthodes connues selon J. March ibid. p. 362.

L'invention a également pour objet les produits II à XIII nouveaux utiles pour la mise en oeuvre du procédé qui vient d'être décrit.

Les exemples suivants illustrent l'invention :

Exemple 1

(suivant le procédé A)

On dissout 130 $cm^3$ (1 mole) de 2-phényl 1-propène dans 1 500 $cm^3$ de chlorure de méthylène. On ajoute 300 $cm^3$ d'eau et 101,5 g (0,5 mole) d'hypochlorite de calcium à 70 % en chlore actif. Sous très forte agitation, on additionne pendant 2 h de la carboglace. On décante les deux phases et sèche la phase organique sur $MgSO_4$. Après évaporation, on obtient 145 g d'une huile jaune. L'analyse RMN (60 MHz) révèle la présence de 45 % de 1-chloro 2-phényl 2-propène, 45 % de 1-chloro 2-phényl 1-propène et 10 % de 1-chloro 2-phényl 2-propanol.

17 g de ce mélange (0,05 molaire en 1-chloro 2-phényl 2-propène) sont dissous dans 100 $cm^3$ de Diméthylformamide. On ajoute 8,2 g (0,05 mole) de benzènesulfinate de sodium et chauffe 2h à 70°C. Le mélange réactionnel est versé dans 350 g de glace et eau. On agite fortement avec 150 $cm^3$ de pentane pour extraire le 1-chloro 2-phényl 1-propène et le 1-chloro 2-phényl 2-propanol qui n'ont pas réagi, pendant 15 à 30 mn jusqu'à cristallisation complète. On filtre les cristaux formés, lave par 50 $cm^3$ de pentane, 50 $cm^3$ de diisopropyléther, essore à fond et sèche. On obtient 9,8 g (76 %) de 2-phényl 1-phénylsulfonyl 2-propène. F = 105°C.

Exemple 2

(suivant le procédé A)

On ajoute 270 $cm^3$ d'une solution 0,79 molaire dans l'éther du complexe 1:1 de cyclopropyllithium/bromure de lithium goutte-à-goutte, à 240 g d'anhydride sulfureux condensé à -70°C.

L'addition terminée, on laisse revenir progressivement à température ambiante sous forte agitation pour éliminer l'anhydride sulfureux en excès. On filtre le solide, lave par 2 x 100 $cm^3$ d'éther, essore à fond et sèche. On obtient 31 g (74 %) d'un solide blanc hydroscopique de cyclopropylsulfinate de lithium (contenant 1 équivalent molaire de bromure de lithium).

10,2 g (0,05 molaire en chlorure) de 1-chloro 2-phényl 2-propène préparé selon l'exemple 1 sont dissous dans 100 $cm^3$ de N-méthyl pyrrolidone. On ajoute 10 g (0,05 molaire en sulfinate) de cyclopropylsulfinate de lithium et chauffe 2 h à 70°C. Le milieu est traité d'une manière identique à l'exemple 1 pour donner 8,9 g de cristaux bruns. Après recristallisation dans l'éthanol on obtient 4,7 g (42 %) de cristaux blancs de 1-cyclopropylsulfonyl 2-phényl 2-propène. F = 76°C.

Exemple 3

(suivant le procédé A)

On charge 11,1 g (0,1 mole) de 2-fluoroaniline et ajoute 300 $cm^3$ d'acide sulfurique à 30 %. On additionne goutte-à-goutte à 0°C, 8,3 g (0,12 mole) de nitrite de sodium en solution dans 40 $cm^3$ d'eau. Le milieu est agité 30 mn à 0°C jusqu'à dissolution totale des sels.

On ajoute 50 $cm^3$ d'acide sulfurique concentré, 150 $cm^3$ d'eau et introduit progressivement à 0°C, 100 g d'anhydride sulfureux. On ajoute alors à 0°C par portions, 10 g de poudre de cuivre activée jusqu'à l'arrêt du dégagement gazeux. La solution est filtrée, le précipité est repris par 200 $cm^3$ d'une solution à 10 % de carbonate de sodium puis refiltré. Le filtrat est acidifié à 0°C par 10 $cm^3$ d'acide sulfurique concentré et réextrait par 2 x 200 $cm^3$ d'éther et évaporé à froid pour donner 4,5 g d'acide sulfinique. On neutralise par 28 $cm^3$ de soude normale et sèche pour obtenir 4,9 g (27 %) de 2-fluorobenzènesulfinate de sodium.

8,24 g (0,027 molaire en chlorure) de 1-chloro 2-phényl 2-propène préparé selon l'exemple 1 sont dissous dans 150 $cm^3$ de Diméthylformamide. On ajoute 4,9 g (0,027 mole) de 2-fluorobenzènesulfinate de sodium

et chauffe 2 h à 70°C. Le milieu est traité d'une manière identique à l'exemple 1 pour donner après recristallisation dans l'éthanol 4,1 g (55 %) de cristaux bruns de 1-(2′-fluorophénylsulfonyl) 2-phényl 2-propène. F = 84°C.

Exemple 4

(suivant le procédé A)

On dissout 15,3 g (0,1 mole) de 2-(3′-chloro 2′-pyridyl) 1-propène dans 200 cm³ de 1,2-dichloroéthane. On ajoute 0,5 g (catalytique) de bis(4-chlorophényl) disélénide et 14,7 g (0,11 mole) de N-chlorosuccinimide et chauffe 24 h à 60°C. On concentre le milieu réactionnel au tiers, filtre le succinimide, et lave la phase organique par 2 x 200 cm³ d'eau, 200 cm³ d'une solution à 15 % de bicarbonate de sodium, 2 x 200 cm³ d'eau et sèche sur $MgSO_4$. Brut : 13,9 g.

L'analyse RMN (60 MHz) révèle la présence de 55 % de 1-chloro 2-(3′-chloro 2′-pyridyl) 2-propène et 45 % de 1-chloro 2-(3′-chloro 2′-pyridyl) 1-propène.

D'une manière identique à la préparation du 2-phényl 1-phénylsulfonyl 2-propène, on traite 7 g de ce mélange (0,02 molaire en 1-chloro 2-(3′-chloro 2′-pyridyl) 2-propène) par le benzènesulfinate de sodium pour obtenir après traitement 4,3 g (73 %) de 2-(3′-chloro 2′-pyridyl) 1-phénylsulfonyl 2-propène. F = 135°C.

Exemple 5

(suivant le procédé B)

4,6 g (0,033 mole) de carbonate de potassium sont mis en suspension dans 100 cm³ d'acétone et l'ensemble est chauffé à 50-60°C. On ajoute sous azote, goutte-à-goutte une solution acétonique (30 cm³) de 4,3 g (0,03 mole) de 2-chlorothiophénol et de 9,1 g (0,03 mole) d'un mélange comprenant 50 % de 1-chloro 2-phényl 2-propène et 50 % de 1-chloro 2-phényl 1-propène. Après 1 h d'agitation à 50-60°C le milieu réactionnel est versé dans 150 g de glace et eau puis réextrait par 3 x 100 cm³ d'éther. On lave par 2 x 150 cm³ d'eau et sèche sur $MgSO_4$. Le brut (11,7 g) est distillé au four à boules pour donner 6,3 g (80 %) de 1-[2′-chlorophénylthio] 2-phényl 2-propène.

$E^{0,5}$ = 180°C. $n_D^{24}$ = 1,6340.

Exemple 6

(suivant procédé B)

1,56 g (0,006 mole) de 1-[2′-chlorophénylthio] 2-phényl 2-propène sont dissous dans 50 cm³ de chlorure de méthylène et refroidis à 0°C. On ajoute 1,9 g (0,006 mole) de mCPBA (acide m-chloro perbenzoïque) à 55 % en solution dans 15 cm³ de chlorure de méthylène goutte-à-goutte en 30 mn. Après 1 h à 0°C la solution est lavée par 75 cm³ de bicarbonate de sodium saturé, 2 x 75 cm³ de saumure et séchée sur $MgSO_4$. Le brut (1,6 g) est chromatographié sur silice pour donner 1,0 g (60 %) de 1-[2′-chlorophénylsulfinyl] 2-phényl 2-propène sous forme d'huile. $n_D^{25}$ = 1,6250.

Exemple 7

(suivant le procédé B)

On traite de la même manière qu'à l'exemple 6, 2,6 g (0,01 mole) de 1-[2′-chlorophénylthio] 2-phényl 2-propène par 6,9 g (0,022 mole) de mCPBA (acide m-chloro perbenzoïque) à 55 % en agitant 2 h à température ambiante. Après le même traitement, le brut (2,6 g) est chromatographié sur silice pour donner 1,4 g (48 %) de 1-[2′-chlorophénylsulfonyl] 2-phényl
2-propène sous forme d'huile. $n_D^{22}$ = 1,6090.

Exemple 8

(suivant le procédé B)

On met en suspension 27 g (0,19 mole) de carbonate de potassium dans 600 cm³ d'acétone. Le milieu est chauffé à 60°C et est dégazé à l'azote. On ajoute goutte-à-goutte à 60°C un mélange de 21,7 g (0,17 mole) de benzylmercaptan et 59 g (0,17 molaire en 1-chloro 2-phényl 2-propène préparé selon l'exemple 4) en solution dans 175 cm³ d'acétone. Le milieu est chauffé 2 h à 60°C.

Le mélange réactionnel est versé dans 2000 g de glace et d'eau, réextrait par 3 x 500 cm³ d'éther. La phase

organique est lavée à l'eau jusqu'à neutralité et séchée sur $MgSO_4$.

L'évaporation laisse 82,5 g d'une huile jaune qui est chromatographiée sur silice (éluant : heptane puis heptane/chloroforme 99/1) pour donner 19,5 g (46 %) de 1-benzylthio 2-phényl 2-propène. Huile incolore. $n_D^{25}$ = 1,6041.

13,2 g (0,055 mole) de 1-benzylthio 2-phényl 2-propène sont dissous dans 300 $cm^3$ de méthanol et 300 $cm^3$ d'eau. On ajoute par portions 36,9 g (0,12 molaire en $KHS0_5$) d'Oxone et agite 3 h à température ambiante. On dilue le mélange réactionnel par 1000 $cm^3$ d'eau, ajoute 100 $cm^3$ de pentane et agite fortement jusqu'à cristallisation. 10,7g (72 %) de cristaux blancs de 1-benzylsulfonyl 2-phényl 2-propène sont obtenus par filtration puis séchage. F = 118°C.

Exemple 9

(suivant le procédé C)

On dissout 6,5 $cm^3$ (0,05 mole) de 2-phényl 1-propène dans 200 $cm^3$ d'acétone. On ajoute 8,2 g (0,05 mole) de benzènesulfinate de sodium. On ajoute ensuite progressivement 12,7 g (0,05 mole) d'iode et l'on laisse agiter 3 jours à température ambiante. L'acétone est évaporée et le résidu repris par 300 $cm^3$ d'éther. On lave par 100 $cm^3$ d'une solution de sulfite de sodium à 10 %, 100 $cm^3$ de bicarbonate de sodium à 5 %, 100 $cm^3$ d'eau et sèche sur $MgSO_4$.

Une chromatographie sur silice fournit 3,1 g (24 %) de 2-phényl 1-phénylsulfonyl 2-propène. F = 106°C.

Exemple 10

(suivant le procédé D)

2,7 g (0,01 mole) d'un mélange 50:50 de 1-[4'-méthylphénylsulfonyl] 2-phényl 1-propène et de 1-[4'-méthylphénylsulfonyl] 2-phényl 2-propène sont dissous dans 10 $cm^3$ d'acétonitrile. On ajoute 3 g (0,02 mole) de 1,8-diazabicyclo [5,4,0] undéc-7-ène et agite 17 h à température ambiante. Le milieu réactionnel est versé dans 100 g d'eau, réextrait par 3 x 50 $cm^3$ d'éther, la phase organique est lavée par 2 x 50 $cm^3$ d'acide chlorhydrique 1N puis 2 x 50 $cm^3$ d'eau et séchée sur $MgSO_4$.

L'analyse RMN (60 MHz) montre un rapport 85:15 de sulfone allylique : vinylique. Une recristallisation du brut (2,4 g) dans l'éther fournit 1,3 g (48 %) de 1-[4'-méthylphénylsulfonyl] 2-phényl 2-propène pur. F = 103°C.

Exemple 11

(suivant le procédé E)

On ajoute 15,6 g (0,1 mole) de méthylphénylsulfone à température ambiante en solution dans 100 $cm^3$ de tétrahydrofuranne, a 100 $cm^3$ d'une solution molaire de bromure d'éthylmagnésium. On agite 1 h jusqu'à la fin du dégagement gazeux. On coule 12,1 g (0,1 mole) de 2-acétylpyridine dans 100 $cm^3$ de THF et agite 3 h à température ambiante. On verse dans 100 $cm^3$ d'eau et 10 g de chlorure d'ammonium et sépare les deux phases. La phase aqueuse est acidifiée, réextraite à l'éther. La phase éthérée est écartée. La phase aqueuse est ramenée à pH basique et réextraite par 3 x 100 $cm^3$ d'éther et séché sur $MgSO_4$.

L'évaporation laisse 16 g de brut qui est chromatographié sur silice (éluant $CH_2Cl_2$ puis $CH_2Cl_2$/acétone 95/5) pour donner 8,3 g (30 %) de cristaux blancs de 1-phénylsulfonyl 2-(2'-pyridyl) 2-propanol. F = 99°C.

On dissout 2,8 g (0,01 mole) de 1-phénylsulfonyl 2-(2'-pyridyl) 2-propanol dans 15 $cm^3$ de pyridine. On ajoute 1,3 g (0,011 mole) de chlorure de thionyle et agite 1 jour à température ambiante. La pyridine est évaporée et le résidu repris par 25 $cm^3$ de chlorure de méthylène, lavé par 3 x 25 $cm^3$ d'eau, séché sur $MgSO_4$.

La solution est filtrée, absorbée sur silice et chromotagraphiée comme précédemment pour donner l'isomère allylique : 1,6 g (62 %) de cristaux de 1-phénylsulfonyl 2-(2'-pyridyl) 2-propène. F = 80°C.

Exemple 12

(suivant le procédé E)

On dissout 14,5 $cm^3$ (0,2 mole) de chlorure de thionyle dans 33,2 $cm^3$ (0,51 mole) d'acide méthanesulfonique et chauffe 1 h au reflux. On refroidit à 25°C, ajoute 15 g (0,1 mole) de 1,3-dichlorobenzène puis 0,9 $cm^3$ (0,01 mole) d'acide trifluorométhanesulfonique et chauffe 3 h à 120°C. On refroidit, verse sur 200 g de glace et d'eau, réextrait par 2 x 150 $cm^3$ d'acétate d'éthyle, lave la phase organique par 3 x 200 $cm^3$ d'eau et sèche sur $MgSO_4$.

Le brut cristallisé (27 g) est recristallisé dans un mélange 1/3 d'acétate d'éthyle/heptane pour donner 11,7

g (51 %) de cristaux blancs de (2,4-dichlorophényl)méthylsulfone. F = 71°C.

D'une manière identique à l'exemple 11, on condense 6 g (0,026 mole) de (2,4-dichlorophényl)méthylsulfone sur 3,2 g (0,026 mole) d'acétophénone pour obtenir après chromatographie sur silice, 2,1 g (23 %) de cristaux blancs 1-(2',4'-dichlorophénylsulfonyl) 2-phényl 2-propanol. F = 94°C.

D'une manière identique à l'exemple 11, on traite 3,2 g (0,009 mole) de 1-(2',4'-dichlorophénylsulfonyl) 2-phényl 2-propanol par 1,1 g (0,009 mole) de chlorure de thionyle dans la pyridine, 30 mn à 20°C. Après traitement et chromatographie sur silice on obtient l'isomère vinylique (1,1 g) et l'isomère allylique : 0,7 g (21 %) d'une huile incolore de 1-(2',4'-dichlorophénylsulfonyl) 2-phényl 2-propène.

Les composés suivants sont préparés suivant les procédés A à E :

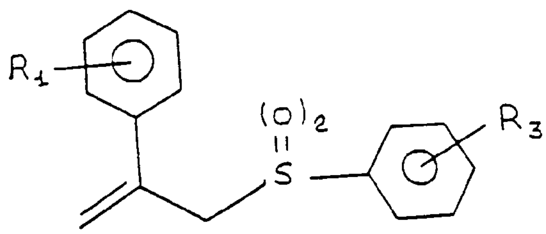

| exemple | $R_1$ | $R_3$ | F (solvant) nD (température) |
|---|---|---|---|
| 13 | 2-F | H | 95°C ($Et_2O$) |
| 14 | 3-F | H | 104,5°C ($Et_2O$) |
| 15 | 4-F | H | 85°C ($CHCl_3$) |
| 16 | 2-Cl | H | 1,5905 (25°C) |
| 17 | 3-Cl | H | 1,6000 (25°C) |
| 18 | 4-Cl | H | 75°C ($Et_2O$) |
| 19 | 4-$CH_3$ | H | 92°C ($Et_2O$) |
| 20 | 4-$OCH_3$ | H | 60°C ($Et_2O$) |
| 21 | 3-$OCH_3$ | H | 1,5920 (25°C) |
| 22 | 3-Br | H | 75°C ($Et_2O$) |
| 23 | 3-$CF_3$ | H | 85°C ($Et_2O$) |
| 24 | 2,4-diCl | H | 85°C ($CCl_4$) |
| 25 | 3,5-diCl | H | 98°C ($Et_2O/CHCl_3$) |
| 26 | 3,4-diCl | H | 85°C ($Et_2O$) |
| 27 | 3-F | 2-Cl | 1,5860 (24°C) |
| 28 | 3-Cl | 2-Cl | 87°C (chromatographie) |
| 29 | 3-F | 2-F | meringue |
| 30 | 3,5-diCl | 2-$CH_3$ | 72°C (EtOH) |
| 31 | 3,5-diCl | 2-Cl | 94°C (EtOH) |
| 32 | 3-$CF_3$ | 2-$CH_3$ | 51°C (EtOH) |
| 33 | 3-$CF_3$ | 2-Cl | 50°C (chromatographie) |
| 34 | H | 2-$OCH_3$ | 1,5910 (23°C) |
| 35 | H | 2-Br | 1,5775 (22°C) |
| 36 | H | 3-F | 70°C ($Et_2O$) |
| 37 | H | 4-F | 83°C (chromatographie) |
| 38 | H | 3-Cl | 71,5°C (chromatographie) |
| 39 | H | 4-Cl | 114°C ($Et_2O/CHCl_3$) |

| | | | |
|---|---|---|---|
| 40 | H | 2-$CH_3$ | 62°C (EtOH) |
| 41 | H | 3-$CH_3$ | 61°C (EtOH) |
| 42 | H | 2,4-di$CH_3$ | 63°C (chromatographie) |
| 43 | H | 2,6-diCl | 1,6125 (25°C) |
| 44 | H | 2-Cl 4-F | 64°C ($Et_2O/CHCl_3$) |
| 45 | H | 4-Br | 133°C (chromatographie) |

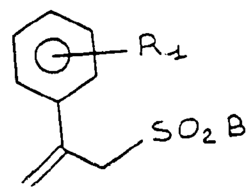

| exemple | $R_1$ | B | F (solvant) nD (température) |
|---|---|---|---|
| 46 | H | $CH_3$ | 1,5695 (25°C) |
| 47 | H | $CH(CH_3)_2$ | 1,55570 (25°C) |
| 48 | H | $(CH_2)_3CH_3$ | 25°C (chromatographie) |
| 49 | 3-Cl | cyclopropyle | 51°C (EtOH) |
| 50 | H | 2-pyridyle | 45°C (chromatographie) |
| 51 | H | cyclopentyle | 1,5675 (23°C) |
| 52 | 3-Cl | $CH(CH_3)_2$ | 1,5715 (23°C) |

Exemple 53

(suivant le procédé F)

5,2 g (0,02 mole) de 2-phényl 1-phénylsulfonyl 2-propène sont dissous dans 50 cm³ de Diméthylsulfoxyde et 10 cm³ d'eau. On ajoute par portion 11,1 g (0,06 mole) de N-bromosuccinimide et agite 4 jours à température ambiante. Le milieu réactionnel est versé dans 350 g de glace et d'eau et est fortement agité avec 100 cm³ de pentane pendant 15 mn. Les cristaux sont filtrés, lavés par 2 x 50 cm³ de pentane et recristallisés dans un mélange éther / chloroforme pour donner 3 g (42 %) de cristaux blancs cotonneux de 1-bromo 2-phényl 3-phénylsulfonyl 2-propanol. F = 91°C.

Exemple 54

(suivant le procédé F)

1,7 g (0,005 mole) de 2-[3'-bromophényl] 1-phénylsulfonyl 2-propène sont dissous dans 50 cm³ d'acétone. On ajoute 0,6 g d'acétate de sodium, 12,5 cm³ d'eau et 12,5 cm³ d'acide acétique. On ajoute par portions 1,4 g (0,01 mole) de N-bromoacétamide et agite 3 h à température ambiante. Le milieu est versé dans 150 cm³ d'eau, réextrait par 3 x 100 cm³ d'éther, la phase organique est lavée jusqu'à neutralité et séchée sur $MgSO_4$. Le brut cristallisé (2,3 g) est recristallisé dans le tétrachlorure de carbone pour donner 0,9 g (40 %) de cristaux

jaunes de 1-bromo 2-[3′-bromophényl] 3-phénylsulfonyl 2-propanol. F = 134°C.

Exemple 55

(suivant le procédé F)

On dissout 2,1 g (0,007 mole) de 1-[2′-bromophénylthio] 2-phényl 2-propène dans 10 $cm^3$ de diméthylsulfoxyde et ajoute 2,5 $cm^3$ d'eau. On ajoute par portions à 0°C, 1,5 g (0,008 mole) de N-bromosuccinimide et agite 1 h 30 à 0°C. Le milieu est versé dans 100 g d'eau, réextrait par 2 x 100 $cm^3$ d'éther et la phase éthérée est lavée, puis séchée sur $MgSO_4$.

Le brut huileux (1,7 g) est précipité au pentane pour donner par filtration 1,4 g (50 %) de cristaux jaunes de 1-bromo 3-[2′-bromophénylthio] 2-phényl 2-propène. F = 65°C.

D'une manière identique à l'exemple 8, on traite 1,1 g (0,003 mole) de 1-bromo 3-[2′-bromophénylthio] 2-phényl 2-propène par 2,5 g d'Oxone pour obtenir après recristallisation dans un mélange 4/1 d'éther/chloroforme, 0,6 g 51 %) de cristaux blancs de 1-bromo 3-[2-bromophénylsulfinyl] 2-phényl 2-propène. F = 134°C.

Exemple 56

(suivant le procédé G)

1,3 g (0,004 mole) de 1-bromo 2-phényl 3-phénylsulfonyl 2-propanol sont dissous dans 30 $cm^3$ d'acétone. On ajoute 0,9 g (0,006 mole) d'iodure de sodium et chauffe 15 h à reflux. Le milieu réactionnel est versé dans 100 $cm^3$ d'eau, réextrait par 3 x 50 $cm^3$ d'éther, lavé par 50 $cm^3$ d'une solution à 15 % de $NaHSO_3$ puis 50 $cm^3$ d'eau et séché sur $MgSO_4$. Le brut cristallisé (0,9 g) est recristallisé dans le tétrachlorure de carbone pour donner 0,5 g (31 %) de cristaux jaunes de 1-iodo 2-phényl 3-phénylsulfonyl 2-propanol. F = 98°C.

Exemple 57

(suivant le procédé H)

A 100 $cm^3$ d'une solution molaire de bromure d'éthylmagnésium, on ajoute 12,5 g (0,08 mole) de méthylphénylsulfone en solution dans 150 $cm^3$ de tétrahydrofuranne à température ambiante. On agite 1 h puis refroidit à -50°C. On coule 12,5 g (0,08 mole) de 2-chloroacétophénone en solution dans 150 $cm^3$ de tétrahydrofuranne et agite 2 h à -20°C. Le milieu est versé dans 200 $cm^3$ d'acide chlorhydrique normal et 6 g de chlorure d'ammonium. On réextrait par 3 x 50 $cm^3$ d'éther, lave jusqu'à neutralité et sèche sur $MgSO_4$. Le brut est précipité à l'heptane et recristallisé dans l'éther isopropylique pour donner 6,5 g (26 %) de cristaux blancs cotonneux de 1-chloro 2-phényl 3-phénylsulfonyl 2-propanol. F = 110°C.

Exemple 58

(suivant le procédé H)

D'une manière identique à l'exemple 57, on traite 7,8 g (0,028 mole) de 2,3′-dibromoacétophénone par 1 équivalent de l'anion magnésium de la méthylphénylsulfone dans le tétrahydrofuranne à -70°C. On agite 2 h à 70°C puis ajoute 7 $cm^3$ d'acide acétique et verse dans 100 g d'eau. On réextrait par 3 x 100 $cm^3$ d'éther, lave par 2 x 100 $cm^3$ de saumure et sèche sur $MgSO_4$. Le brut est précipité par 50 $cm^3$ d'éther à chaud et recristallisé dans l'éther isopropylique pour donner 2,4 g (20 %) de cristaux blancs de 1-bromo 2-[3′-bromophényl] 3-phénylsulfonyl 2-propanol. F = 132°C.

Exemple 59

(suivant le procédé I)

On dissout 0,8 g (0,0025 mole) de 2-[2′-chlorophénylsulfonylméthyl] 2-[3′-fluorophényl] oxiranne dans 10 $cm^3$ de tétrahydrofuranne. On ajoute à 0°C, 10 $cm^3$ d'une solution 0,4 molaire de $Li_2NiBr_4$ et agite 2 h à température ambiante. On ajoute 12 $cm^3$ d'une solution tampon de pH égal à 7, réextrait par 3 x 50 $cm^3$ de chlorure de méthylène et sèche sur $MgSO_4$. L'évaporation laisse 1,0 g d'une huile qui est chromatographiée sur silice (éluant : heptane/chloroforme 80/20) pour donner 0,7 g (68 %) de cristaux blancs de 1-bromo 3-[2′-chlorophénylsulfonyl] 2-[3′-fluorophényl] 2-propanol. F = 121°C.

Exemple 60

(suivant le procédé J)

On met en suspension 2,6 g (0,01 mole) de 2-phényl 1-phénylsulfonyl 2-propène dans 50 $cm^3$ d'acide acétique. On ajoute alors 4,1 g (0,05 mole) d'acétate de sodium puis 3,6 g (0,02 mole) de N-bromosuccinimide et agite 3 h à température ambiante. Le milieu est alors versé dans 200 g de glace et d'eau et est fortement agité jusqu'à formation d'une gomme. La gomme est filtrée, redissoute dans 50 $cm^3$ de chlorure de méthylène et est lavée par 100 $cm^3$ d'une solution à 10 % de bicarbonate de sodium, 2 x 100 $cm^3$ d'eau et séchée sur $MgSO_4$. L'évaporation laisse 3,5 g d'une huile jaune. Cette huile est dissoute dans 10 $cm^3$ d'une mélange éther/chloroforme et stockée à -18°C pendant 1 nuit.

Après filtration on obtient 2,3 g (59 %) de cristaux jaunes de 2-acétoxy 1-bromo 2-phényl 3-phénylsulfonylpropane.
F = 103°C.

Exemple 61

(suivant le procédé J)

D'une manière identique à l'exemple 60, on traite 2,6 g (0,01 mole) de 2-phényl 1-phénylsulfonyl 2-propène par 1,8 g (0,01 mole) de N-bromosuccinimide dans l'acide trifluoroacétique en présence de 6,8 g (0,05 mole) de trifluoroacétate de sodium. Après traitement, l'évaporation laisse 4 g d'une huile qui est cristallisée dans 10 $cm^3$ d'éther à -18°C pendant 1 nuit. On filtre 2,1 g (47 %) de cristaux blancs de 1-bromo 2-trifluoroacétoxy 2-phényl 3-phénylsulfonylpropane. F = 100°C.

Exemple 62

(suivant le procédé K)

5,16 g (0,02 mole) de 2-phényl 1-phénylsulfonyl 2-propène sont dissous dans 40 $cm^3$ d'acétone. On ajoute 1,3 g (0,005 mole) d'acétate de tétrabutylammonium et 4,4 $cm^3$ (0,03 mole) d'hydroperoxyde de tertiobutyle à 70 % et agite 15 h à température ambiante.

On ajoute 80 $cm^3$ d'éther, refroidit à 0°C puis ajoute 10 $cm^3$ d'une solution à 10 % de $NaHSO_3$ en une seule fois. Après 1 h d'agitation on décante les deux phases, sature la phase aqueuse par 4 g de NaCl, réextrait par 2 x 25 $cm^3$ d'éther et lave l'ensemble des phases organiques par 50 $cm^3$ de saumure et sèche sur $MgSO_4$.

Le brut (5,5 g) cristallisé est recristallisé dans l'acétone pour donner 3,6 g (61 %) de cristaux blancs de 2-phényl 3-phénylsulfonyl 1,2-propanediol. F = 137°C.

1,3 g (0,004 mole) de 2-phényl 3-phénylsulfonyl 1,2-propanediol sont dissous dans 25 $cm^3$ de pyridine et refroidit à 0°C. On ajoute 0,5 g (0,005 mole) de chlorure de méthanesulfonyle et agite 2 h à température ambiante. Le milieu est versé dans un mélange de 100 g de glace et 30 $cm^3$ d'acide chlorhydrique à 35 % et est fortement agité avec 25 $cm^3$ de pentane. Les cristaux formés sont filtrés, séchés et recristallisés dans l'acétate d'éthyle pour donner 0,7 g (42 %) de cristaux blancs de 1-méthanesulfonate 2-phényl 3-phénylsulfonyl 2-propanol. F = 144°C.

Exemple 63

(suivant le procédé L)

1,1 g (0,003 mole) de 1-bromo 2-phényl 3-phénylsulfonyl 2-propanol sont dissous dans 20 $cm^3$ de diméthoxyéthane. On ajoute 1,4 g (0,006 mole) d'oxyde d'argent $Ag_2O$ et chauffe 20 h au reflux. Le mélange est filtré à chaud sur supercel, le gateau est lavé par 100 $cm^3$ de chlorure de méthylène. La phase organique est séchée sur $MgSO_4$.

Le brut cristallisé (0,9 g) est recristallisé dans le tétrachlorure de carbone pour donner 0,7 g (85 %) de cristaux jaunes de 2-phényl 2-[phénylsulfonylméthyl] oxiranne. F = 89°C.

Exemple 64

(suivant le procédé N)

D'une manière identique à l'exemple 62, 10,3 g (0,033 mole) de 2-[3'-fluorophényl] 1-[2'-chlorophénylsulfonyl] 2-propène sont traités par 7,5 $cm^3$ (0,05 mole) d'hydroperoxyde de tertiobutyle à 70 % en présence de 1,1 g (0,008 mole) d'acétate de tétrabutylammonium et d'oxyde d'osmium catalytique. Après traitement et recristallisation dans l'acétone on obtient 8,7 g (76 %) de cristaux blancs de 3-[2'-chlorophénylsulfonyl] 2-[3'-

fluorophényl] 1,2-propanediol. F = 132°C.

4 g (0,012 mole) de 3-[2'-chlorophénylsulfonyl] 2-[3'-fluorophényl] 1,2-propanediol sont dissous dans 75 cm³ de chloroforme. On refroidit à 0°C et ajoute 3,2 g (0,012 mole) de triphénylphosphine puis goutte-à-goutte 2,8 g (0,016 mole) de diéthylazodicarboxylate. Le milieu est agité 15 h à température ambiante. Le chloroforme est évaporé et le résidu chromatographié sur silice (éluant heptane/chloroforme 50/50) pour donner 2,4 g (61 %) de cristaux blancs de 2-[2'-chlorophénylsulfonylméthyl] 2-[3'-fluorophényl] oxiranne. F = 104°C.

Les composés rassemblés dans les tableaux ci-après ont été préparés selon les méthodes F à I et K.

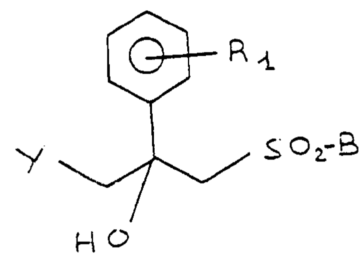

| exemple | Y | $R_1$ | B | F (solvant) |
|---|---|---|---|---|
| 65 | Br | 3,5-diCl | phényle | 131°C ($ET_2O/CHCl_3$ |
| 66 | Br | H | 2-Cl phényle | 140°C (chromato.) |
| 67 | Br | H | 2-F phényle | 117°C ($Et_2O/CHCl_3$) |
| 68 | Br | H | 2-$CH_3$ phényle | 123°C ($Et_2O/CHCl_3$) |
| 69 | Cl | H | 2-Cl phényle | 131°C ($CH_2Cl_2/iPr_2O$) |
| 70 | Br | H | 3-$CH_3$ phényle | 122°C (EtOH) |
| 71 | Br | H | 2-Cl4-F phényle | 122°C ($Et_2O/CHCl_3$) |
| 72 | Br | H | $CH(CH_3)_2$ | 101°C ($CHCl_3$) |
| 73 | Cl | H | $CH(CH_3)_2$ | 110°C ($iPr_2O$) |
| 74 | Br | 3-Cl | $CH(CH_3)_3$ | 83°C ($Et_2O/CHCl_3$) |
| 75 | Br | H | benzyle | 91°C (chromato.) |
| 76 | Br | H | cyclopentyle | meringue |
| 77 | Br | H | cyclopropyle | 71°C ($Et_2O/CHCl_3$) |

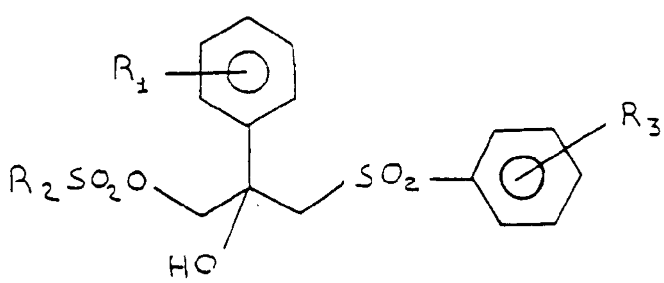

| exemple | $R_1$ | $R_2$ | $R_3$ | F (solvant) |
|---|---|---|---|---|
| 78 | H | $CH_3(CH_2)_2$ | H | 112°C (AcOEt/Pentane) |
| 79 | H | $(CH_3)_2CH$ | H | 83°C (AcOEt) |
| 80 | H | $Cl(CH_2)_3$ | H | 98°C (AcOEt/Pentane) |
| 81 | 3-Br | $CH_3$ | H | 147°C ($CH_2Cl_2$) |
| 82 | 3-F | $CH_3$ | 2-Cl | 123°C (AcOEt) |
| 83 | H | $CH_3$ | 2-$CH_3$ | 160°C (AcOEt) |
| 84 | H | 4-Br phényle | H | 118°C |
| 85 | H | 4-$OCH_3$ phényle | H | 129°C (AcOEt/Pentane) |
| 86 | H | 4-$NO_2$ phényle | H | 116°C (AcOEt/Pentane) |
| 87 | H | 4-$CH_3$ phényle | H | 114°C (AcOEt) |

L'invention concerne également l'utilisation à titre d'herbicide des composés de formule (I). Comme mauvaises herbes pouvant être contrôlées ou détruites par les composés de formule (I), on peut citer :

Graminées / Cypéracées

| Abreviations | Nom latin | Nom français |
|---|---|---|
| AVE | Avena fatua | Folle avoine |
| ECH | Echinochloa crusgalli | Panisse |
| LOL | Lolium multiflorum | Ray-grass |
| SOR | Sorghum halepense | Sorgho d'Alep |
| ALO | Alopecurus myosuroïdes | Sorgho d'Alep |
| CYP | Cyperus esculentus | Cyperus |
| DIG | Digitaria sanguinalis | Digitaire |
| PAN | Panicum miliaceum | Panic |
| SET | Setaria faberié | Millet |

Dicotylédones :

| Abreviations | Nom latin | Nom français |
|---|---|---|
| IPO | Ipomea purpurea | Liseron pourpre |
| SIN | Sinapis arvensis | Moutarde |
| ABU | Abutilon theophrasti | Abutilon |
| SOL | Solanum nigrum | Morelle noire |

L'utilisation des composés de formule (I) est la plupart du temps sous forme de composition herbicide comportant un ou plusieurs supports acceptables en agriculture.

En effet pour leur emploi pratique, les composés selon l'invention sont rarement utilisés seuls. Le plus souvent ces composés font partie de compositions. Ces compositions, utilisables comme agents herbicides, contiennent comme matière active un composé selon l'invention tel que décrit précédemment en mélange avec les supports solides ou liquides, acceptables en agriculture et les agents tensio-actifs également acceptables en agriculture. En particulier sont utilisables les supports inertes et usuels et les agents tensio-actifs usuels. Ces compositions font également partie de l'invention.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... . Plus généralement les composés utilisés dans l'invention peuvent être combinés à tous les additifs solides ou liquides correspondant aux techniques habituelles de la mise en formulation.

D'une façon générale, les compositions selon l'invention contiennent habituellement de 0,05 à 95 % environ (en poids) d'un composé selon l'invention, un ou plusieurs supports solides ou liquides et, éventuellement, un ou plusieurs agents tensioactifs.

Par le terme "support", dans le présent exposé, on désigne une matière organique ou minérale, naturelle ou synthétique, avec laquelle le composé est combiné pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau ; alcools, notamment le butanol etc..).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque le composé et/ou le support inerte ne sont pas solubles dans l'eau et que l'agent vecteur de l'application est l'eau.

Ainsi donc, les compositions à usage agricole selon l'invention peuvent contenir les matières actives selon l'invention dans de très larges limites, allant de $5.10^{-5}$% à 95 % (en poids). Leur teneur en agent tensio-actif est avantageusement comprise entre 5 % et 40 % en poids.

Ces compositions selon l'invention sont elles-mêmes sous des formes assez diverses, solides ou liquides.

Comme formes de compositions solides, on peut citer les poudres pour poudrage (à teneur en composé pouvant aller jusqu'à 100 %) et les granulés, notamment ceux obtenus par extrusion, par compactage, par imprégnation d'un support granulé, par granulation à partir d'une poudre (la teneur en composé dans ces granulés étant entre 0,5 et 80 % pour ces derniers cas).

Les poudres mouillables (ou poudre à pulvériser) sont habituellement préparées de manière qu'elles contiennent 20 à 95 % de matière active, et elles contiennent habituellement, en plus du support solide, de 0 à 30 % d'un agent mouillant, de 3 à 20 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

Pour obtenir les poudres à pulvériser ou poudres mouillables, on mélange intimement les matières actives dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

A titre d'exemple, voici diverses compositions de poudres mouillables (ou poudres à pulvériser) :

Exemple F 1

- matière active (composé n° 1) .................. 50 %
- alcool gras éthoxylé (agent mouillant)......... 2,5 %
- phényléthylphénol éthoxylé (agent dispersant).. 5 %
- craie (support inerte) ........................42,5 %

Exemple F 2 :

- matière active (composé n° 1) .................. 10 %
- alcool synthétique oxo de type ramifié, en $C_{13}$ éthoxylé par 8 à 10 oxyde d'éthylène (agent mouillant) ....................................0,75 %
- lignosulfonate de calcium neutre (agent dispersant ........................................ 12 %
- carbonate de calcium (charge inerte) ....q.s.p. 100 %

Exemple F 3 : cette poudre mouillable contient les mêmes ingrédients que dans l'exemple précédent, dans les proportions ci-après :

- matière active ................................ 75 %
- agent mouillant ..............................1,50 %
- agent dispersant ................................8 %
- carbonate de calcium (charge inerte) ....q.s.p. 100 %

Exemple F 4 :

- matière active (composé n° 1) .................. 90 %
- alcool gras éthoxylé (agent mouillant ........... 4 %
- phényléthylphénol éthoxylé (agent dispersant) ... 6 %

Exemple F 5 :

- matière active (composé n° 1) .................. 50 %
- mélange de tensio-actifs anioniques et non ioniques (agent mouillant) ..................... 2,5 %
- lignosulfonate de sodium (agent dispersant) .... 5 %
- argile kaolinique (support inerte) .............42,5 %

Les composés selon l'invention peuvent être formulés sous la forme de granulés dispersibles dans l'eau également compris dans le cadre de l'invention.

Ces granulés dispersibles, de densité apparente généralement comprise entre environ 0,3 et 0,6, ont une dimension de particules généralement comprise entre environ 150 et 2000 et de préférence entre 300 et 1500 microns.

La teneur en matière active de ces granulés est généralement comprise entre environ 1 % et 90 %, et de

préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensio-actifs (à raison de 2 à 20 % en poids du granulé) dont plus de la moitié est, par exemple, constituée par au moins un agent dispersant, essentiellement anionique, tel qu'un polynaphtalène sulfonate alcalin ou alcalino terreux ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc..). On termine généralement par un concassage suivi d'un tamisage à la dimension de particule choisie dans les limites mentionnées ci-dessus.

De préférence, il est obtenu par extrusion, en opérant comme indiqué dans les exemples ci-après.

Exemple F 6 : Granulés dispersibles

Dans un mélangeur, on mélange 90 % en poids de matière active (composé n° 1) et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

Exemple F 7 : Granulés dispersibles

Dans un mélangeur, on mélange les constituants suivants :

- matière active (composé n° 1) .................. 75 %
- agent mouillant (alkylnaphtalène sulfonate de sodium ........................................ 2 %
- agent dispersant (polynaphtalène sulfonate de sodium) ...................................... 8 %
- charge inerte insoluble dans l'eau (kaolin) ..... 15 %

Ce mélange est granulé en lit fluide, en présence d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,001 à 20 % de matière active.

En plus du solvant, les concentrés émulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précédemment cités.

A partir de ces concentrés, on peut obtenir par dilution avec de l'eau des émulsions de toute concentration désirée, qui conviennent particulièrement à l'application sur les cultures. A titre d'exemple, voici la composition de quelques concentrés émulsionnables :

Exemple F 8 :

| | | |
|---|---|---|
| - matière active | | 400 g/l |
| - dodécylbenzène sulfonate alcalin | | 24 g/l |
| - nonylphénol oxyéthylé à 10 molécules d'oxyde d'éthylène | | 16 g/l |
| - cyclohexanone | | 200 g/l |
| - solvant aromatique | q.s.p | 1 litre |

Selon une autre formule de concentré émulsionnable, on utilise :

Exemple F 9 :

| | |
|---|---|
| - matière active | 250 g |
| - huile végétale époxydée | 25 g |
| - mélange de sulfonate d'alcoylaryle et d'éther de polyglycol et d'alcools gras | 100 g |
| - diméthylformamide | 50 g |
| - xylène | 575 g |

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici une composition de suspension concentrée :

Exemple F 10 :

| | |
|---|---|
| - composé | 500 g |
| - phosphate de tristyrylphénol polyéthoxylé | 50 g |
| - alkylphénol polyéthoxylé | 50 g |
| - polycarboxylate de sodium | 20 g |
| - éthylène glycol | 50 g |
| - huile organopolysiloxanique (antimousse) | 1 g |
| - polysaccharide | 1,5 g |
| - eau | 316,5 g |

Les dispersions et émulsions aqueuses, par exemple les compositions obtenues en diluant à l'aide d'eau

une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

En ce qui concerne les compositions adaptées au stockage et au transport, elles contiennent plus avantageusement de 0,5 à 95 % (en poids) de substance active.

La présente invention concerne aussi un procédé de désherbage (notamment de zones de culture dicotylédones ou de maïs) qui consiste à appliquer aux plantes devant être détruites une quantité efficace d'un composé de formule (I).

Les produits et compositions selon l'invention s'appliquent commodément sur la végétation et notamment sur les mauvaises herbes à éliminer lorsque celles-ci présentent un feuillage vert.

Néanmoins on utilisera, de préférence, un procédé de désherbage qui consiste à appliquer une quantité efficace d'un composé de formule (I) sur des zones ou terrains où l'on veut empêcher la pousse ou le développement de plantes n'ayant pas encore poussé (application de préémergence).

On peut opérer de manière à ce que la culture soit semée avant ou après traitement.

La dose d'application de matière active est généralement comprise entre 1 et 8 000 g/ha.

Les exemples ci-desous illustrent l'invention :

Exemple A - Application herbicide, en prélevée des espèces végétales

Dans des pots de 7 x 7 x 8 cm remplis de terre agricole légère, on sème un nombre de graines déterminé en fonction de l'espèce végétale et de la grosseur de la graine.

Les pots sont traités par pulvérisation de bouillie en quantité correspondant à une dose volumique d'application de 500 l/ha et contenant la matière active à la concentration désirée.

Le traitement avec la bouillie est donc effectué sur des graines non recouvertes de terre (on utilise le terme de bouillie pour désigner, en général, les compositions diluées à l'eau, telles qu'on les applique sur les végétaux).

La bouillie utilisée pour le traitement est une solution ou suspension de la matière active dans un mélange acétone/eau en proportions 50/50, en présence de 0,05 % en poids de Cemulsol NP 10 (agent tensio-actif) constitué d'alkylphénol polyéthoxylé, notamment de nonylphénol polyéthoxylé) et 0,04 % en poids de tween 20 (agent tensio-actif constitué d'un oléate de dérivé polyoxyéthyléné du sorbitol).

Dans le cas d'une suspension, celle-ci est obtenue en mélangeant et broyant les ingrédients dans un microniseur de façon à obtenir une grosseur moyenne de particules inférieure à 40 microns.

Après traitement, on recouvre les graines d'une couche de terre d'environ 3 mm d'épaisseur.

Les pots sont alors placés dans des bacs destinés à recevoir l'eau d'arrosage, en subirrigation, et maintenus pendant 24 jours à température ambiante sous 70 % d'humidité relative.

Notation de l'activité herbicide

Le relevé est effectué de la façon suivante :

Au bout de 24 jours, on mesure un pourcentage (D) de destruction du nombre de pieds dans le pot traité par rapport au nombre de plantes dans les pots non traités (témoin). On mesure, sur les plantes traitées restantes, le pourcentage de réduction de taille (RT) par rapport aux plantes témoin.

Le pourcentage de volume foliaire non détruit par le produit est donc donné par la formule

$$\frac{[100 - D] \times [100 - RT]}{100} = A$$

Cette valeur A est transformée en notation de 0 à 5 selon l'échelle suivante :

| | notation | |
|---|---|---|
| $0 \leq A < 10$ | 5 | (destruction complète) |
| $10 \leq A < 30$ | 4 | |
| $30 \leq A < 50$ | 3 | |
| $50 \leq A < 70$ | 2 | |
| $70 \leq A < 90$ | 1 | |
| $90 \leq A \leq 100$ | 0 | (pas d'effet) |

Les résultats obtenus sont présentés après l'exemple B pour des doses d'application de 4000 g/ha.

Exemple B - Application herbicide, en postlevée des espèces végétales.

Dans des pots de 7 x 7 x 8 cm remplis de terre agricole légère, on sème un nombre de graines déterminé en fonction de l'espèce végétale et de la grosseur de la graine.

On recouvre ensuite les graines d'une couche de terre d'environ 3 mm d'épaisseur et on laisse germer la graine jusqu'à ce qu'elle donne naissance à une plantule au stade convenable. Le stade de traitement pour les graminées est le stade "deuxième feuille en formation". Le stade de traitement pour les dicotylédones, est le stade "cotylédons étalés, première feuille vraie en développement".

Les pots sont alors traités par pulvérisation de bouillie en quantité correspondant à une dose volumique d'application de 500 l/ha et contenant la matière active à la concentration désirée.

La bouillie a été préparée de la même manière qu'à l'exemple A.

Les pots traités sont ensuite placés dans des bacs destinés à recevoir l'eau d'arrosage, en subirrigation, et maintenus pendant 24 jours à température ambiante sous 70 % d'humidité relative.

Notation de l'activité herbicide

Le relevé est effectué de la façon suivante :

Au bout de 24 jours, on mesure un pourcentage (D) de destruction du nombre de pieds dans le pot traité par rapport au nombre de plantes dans les pots non traités (témoin). On mesure, sur les plantes traitées restantes, le pourcentage de réduction de taille (RT) par rapport aux plantes témoin.

Le pourcentage de volume foliaire non détruit par le produit est donc donné par la formule

$$\frac{[100 - D] \times [100 - RT]}{100} = A$$

Cette valeur A est transformée en notation de 0 à 5 selon l'échelle suivante :

| | notation | |
|---|---|---|
| $0 \leq A < 10$ | 5 | (destruction complète) |
| $10 \leq A < 30$ | 4 | |
| $30 \leq A < 50$ | 3 | |
| $50 \leq A < 70$ | 2 | |
| $70 \leq A < 90$ | 1 | |
| $90 \leq A \leq 100$ | 0 | (pas d'effet) |

Les résultats obtenus sont présentés après l'exemple B pour des doses d'application de 4000 g/ha.

Les espèces végétales utilisées dans ces exemples A et B sont :

| Abreviations | Nom latin | Nom français |
|---|---|---|
| AVE | Avena fatua | Folle avoine |
| ECH | Echinochloa crusgalli | Panisse |
| LOL | Lolium multiflorum | Ray-grass |
| CYP | Cyperus esculentus | Cyperus |
| DIG | Digitaria sanguinalis | Digitaire |
| ALO | Alopecurus myosuroides | Vulpin |

| COMPOSE N° | ACTIVITE HERBICIDE DE PRELEVEE Dose : 4 Kg m.a./ha | | | | | |
|---|---|---|---|---|---|---|
| | AVE | ECH | LOL | DIG | CYP | ALO |
| 3 | 5 | 5 | 5 | 5 | 5 | - |
| 4 | 3 | 5 | 4 | 5 | 5 | - |
| 6 | 5 | 5 | 5 | 5 | 5 | - |
| 8 | 0 | 4 | 2 | 5 | 0 | - |
| 14 | 5 | 5 | 5 | 5 | 3 | - |
| 17 | 5 | 5 | 5 | 5 | 5 | - |
| 22 | 5 | 4 | 5 | 3 | 0 | - |
| 23 | 5 | 5 | 5 | 3 | 1 | - |
| 27 | 3 | 5 | 5 | 5 | 4 | - |
| 28 | 5 | 4 | 5 | 4 | 3 | - |
| 29 | 5 | 5 | - | 5 | 4 | 5 |
| 37 | 4 | 5 | 5 | 5 | 0 | - |
| 40 | 3 | 5 | 5 | 5 | 3 | - |
| 41 | 1 | 4 | - | 5 | 1 | 5 |
| 42 | 5 | 5 | 5 | 5 | 4 | - |
| 44 | 5 | 5 | - | 5 | 4 | 5 |
| 46 | 0 | 3 | 4 | 4 | 0 | - |
| 47 | 5 | 5 | 5 | 5 | 1 | - |
| 52 | 5 | 5 | 5 | 5 | 5 | - |
| 53 | 5 | 5 | 5 | 5 | 4 | - |
| 54 | 5 | 5 | 5 | 5 | 5 | - |
| 56 | 5 | 5 | 5 | 5 | 4 | - |
| 59 | 5 | 5 | - | 5 | 5 | 5 |
| 60 | 2 | 5 | - | 5 | 3 | 4 |
| 61 | 5 | 5 | - | 5 | 4 | 5 |
| 62 | 5 | 5 | 5 | 5 | 3 | - |
| 63 | 4 | 4 | 3 | 3 | 0 | - |
| 65 | 5 | 5 | 5 | 5 | 5 | - |
| 66 | 3 | 5 | - | 5 | 3 | 5 |
| 67 | 4 | 5 | - | 5 | 3 | 5 |
| 69 | 5 | 5 | 5 | 5 | 4 | - |

| | | | | | | |
|---|---|---|---|---|---|---|
| 70 | 5 | 5 | - | 5 | 4 | 5 |
| 72 | 5 | 5 | 5 | 5 | 5 | - |
| 73 | 4 | 3 | 5 | 4 | 5 | - |
| 81 | 5 | 5 | 5 | 5 | 5 | - |
| 82 | 5 | 5 | - | 5 | 5 | 5 |

| COMPOSE N° | ACTIVITE HERBICIDE DE POST LEVEE Dose : 4 Kg m.a./ha | | | | |
|---|---|---|---|---|---|
| | AVE | ECH | LOL | DIG | ALO |
| 3 | 0 | 4 | 3 | 4 | - |
| 6 | 2 | 3 | 2 | 4 | - |
| 17 | 4 | 3 | 3 | 1 | - |
| 23 | 3 | 3 | 2 | 0 | - |
| 27 | 0 | 2 | 3 | 3 | - |
| 28 | 3 | 4 | 4 | 2 | - |
| 40 | 1 | 4 | 3 | 3 | - |
| 42 | 0 | 4 | - | 3 | 4 |
| 44 | 3 | 4 | - | 4 | 4 |
| 52 | 1 | 3 | 4 | 3 | - |
| 53 | 0 | 4 | 2 | 3 | - |
| 54 | 1 | 3 | 3 | 1 | - |
| 56 | 0 | 4 | 3 | 3 | - |
| 59 | 0 | 4 | - | 4 | 3 |
| 60 | 0 | 4 | - | 2 | 4 |
| 63 | 0 | 4 | 2 | 3 | - |
| 65 | 0 | 3 | 3 | 3 | - |
| 72 | 4 | 3 | 4 | 2 | - |
| 81 | 0 | 4 | 4 | 3 | - |
| 82 | 0 | 4 | - | 2 | 4 |

Exemple C - Essai de sélectivité en grandes cultures avec application herbicide, en prelevée des espèces végétales.

Dans des pots de 7 x 7 x 8 cm remplis de terre agricole légère, on sème un nombre de graines déterminé en fonction de l'espèce végétale et de la grosseur de la graine.

On recouvre ensuite les graines d'une couche de terre d'environ 3 mm d'épaisseur.

Les pots sont alors traités par pulvérisation de bouillie en quantité correspondant à une dose volumique d'application de 500 l/ha et contenant la matière active à la concentration désirée.

La bouillie a été préparée de la même manière qu'à l'exemple A.

Les pots traités sont ensuite placés dans des bacs destinés à recevoir l'eau d'arrosage, en subirrigation, et maintenus pendant 24 jours à température ambiante sous 70 % d'humidité relative.

Notation de l'activité herbicide

Le relevé est effectué de la façon suivante :

Au bout de 24 jours, on mesure un pourcentage (D) de destruction du nombre de pieds dans le pot traité par rapport au nombre de plantes dans les pots non traités (témoin). On mesure, sur les plantes traitées restantes, le pourcentage de réduction de taille (RT) par rapport aux plantes témoin.

Le pourcentage de volume foliaire non détruit par le produit est donc donné par la formule :

$$\frac{[100-D]\times[100-RT]}{100} = A$$

Cette valeur A est transformée en notation de 0 à 5 selon l'échelle suivante :

| | | | notation |
|---|---|---|---|
| 0 | A | 10 | 5 (destruction complète) |
| 10 | A | 30 | 4 |
| 30 | A | 50 | 3 |
| 50 | A | 70 | 2 |
| 70 | A | 90 | 1 |
| 90 | A | 100 | 0 (pas d'effet) |

Ainsi, un produit est jugé comme sélectif vis à vis de la culture lorsque la valeur A notée est de 0 ou 1.

Les résultats obtenus sont présentés dans l'exemple C pour des doses d'application de 1 ou 2 ou 4 kg de matière active par hectare selon les produits.

Les espèces végétales utilisées dans cet exemple sont :

1) Pour les adventices

| Abréviations | Nom latin | Nom français |
|---|---|---|
| ECH | Echinochloa crus-galli | Panisse |
| PAN | Panicum miliaceum | Panic |
| DIG | Digitaria sanguinalis | Digitaire |
| SOR | Sorghum halepense | Sorgho d'Alep |
| SET | Setaria faberii | Millet |
| CYP | Cyperus esculantus | Cyperus |

2) Pour les cultures

| Abréviations | Nom latin | Nom français |
|---|---|---|
| TRZ | Triticum aestivum | Blé |
| ZEA | Zea mays | Maïs |
| ORY | Oryza sativa | Riz |
| GLX | Glycine maximum | Soja |

| COMPOSE N° | Dose appliquée (Kg/ha) | ECH | PAN | DIG | SOR | SET | CYP | TRZ | ZEA | ORY | GLX |
|---|---|---|---|---|---|---|---|---|---|---|---|
| | | ESSAI DE SELECTIVITE EN GRANDES CULTURES | | | | | | | | | |
| | | ACTIVITE HERBICIDE DE PRELEVEE | | | | | | | | | |
| 6 | 2 | 5 | 4 | 5 | 5 | 5 | 5 | 1 | 0 | 0 | 0 |
| 52 | 4 | 5 | 5 | 5 | 3 | 5 | 0 | 2 | 2 | 2 | 0 |
| 3 | 2 | 3 | 5 | 5 | 5 | 5 | 1 | 0 | 1 | 2 | 0 |
| 40 | 1 | 4 | 5 | 5 | 3 | 4 | 1 | 0 | 1 | 1 | 0 |
| 54 | 1 | 5 | 5 | 5 | 4 | 5 | 2 | 0 | 0 | 1 | 0 |
| 28 | 2 | 5 | 5 | 5 | 2 | 5 | 4 | 0 | 0 | 0 | 0 |
| 27 | 2 | 5 | 5 | 5 | 4 | 5 | - | 0 | 0 | 1 | 0 |
| 44 | 2 | 5 | 5 | 5 | 3 | 5 | - | 0 | 0 | 3 | 2 |

Comme on peut le voir sur le tableau de résultats de cet exemple C, de nombreux produits présentent une excellente activité antigramninée de prélevée tout en montrant une excellente sélectivité pour 1 ou 2 ou 3 ou 4 des 4 cultures testées = blé, maïs, riz, soja.

## Revendications

### Revendications pour les Etats contractants suivants : AT, BE, CH, DE, FR, GB, IT, LI, LU, NL, SE

**1.** Composés de formule :

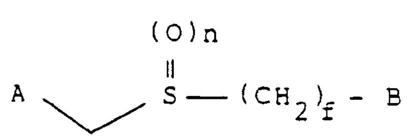

I

dans laquelle :
n = 0, 1, 2

f = 0, 1

A est choisi parmi les groupes

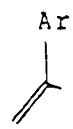 (A1) 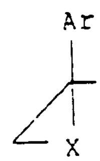 (A2) 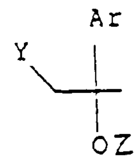 (A3)

dans lesquels :

Ar est choisi parmi les groupes

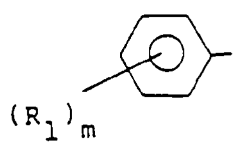
Ar-1

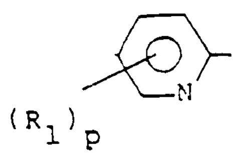
Ar-2

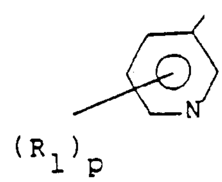
Ar-3

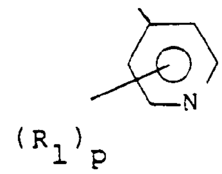
Ar-4

X étant un atome d'oxygène ou de soufre,

$R_1$ étant un atome d'halogène (notamment Cl ou Br ou F), un groupe $C_1$-$C_4$ alkyle, $C_1$-$C_4$ alkoxy, $C_1$-$C_4$ haloalkyle, $C_1$-$C_4$ haloalkoxy, nitro, cyano, $C_6$-$C_{10}$ aryle (notamment phényle ou naphtyle), $C_7$-$C_{11}$ aralkyle (notamment benzyle), $C_6$-$C_{10}$ aryloxy (notamment phénoxy ou napthoxy) éventuellement substitué par 1 ou 2 atomes d'halogène, $C_7$-$C_{11}$ aralkyloxy (notamment benzyloxy) éventuellement substitué par 1 ou 2 atomes d'halogène,

m = 0, 1, 2, 3, 4, 5

p = 0, 1, 2, 3, 4

les différents radicaux $R_1$ étant identiques ou différents lorsque m ou p est supérieur ou égal à 2.

Y est un atome de chlore ou de brome ou d'iode ou un groupement $OSO_2R_2$,

$R_2$ étant un groupement $C_1$-$C_6$ alkyle, $C_6$-$C_{10}$ aryle, $C_7$-$C_{11}$ aralkyle les dits groupements étant éventuellement substitués par 1 à 3 halogènes ou groupements $C_1$-$C_4$ alkyle, C-$C_4$ alkoxy, $C_1$-$C_4$ haloalkyle, $C_1$-$C_4$ haloalkoxy, nitro,

Z étant un atome d'hydrogène ou un groupe $(C{=}O)R_{11}$, $R_{11}$ est hydrogène, $C_1$-$C_4$ alkyle, $C_1$-$C_4$ haloalkyle,

B est choisi parmi les groupes $C_1$-$C_{10}$ alkyle,

$C_3$-$C_{10}$ cycloalkyle, ces groupes étant éventuellement substitués par 1 à 6 atomes d'halogène ou choisi parmi les groupes

B1 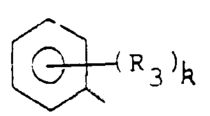 ; B2 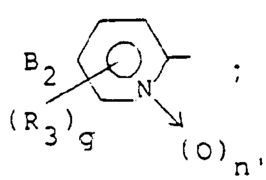 ; B3 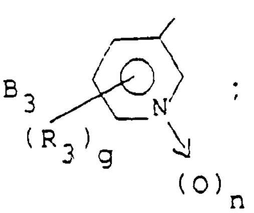 ; B4 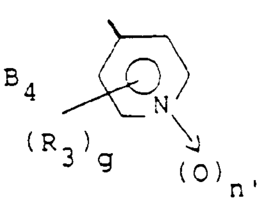

$R_3$ ayant l'une des significations indiquées pour $R_1$ ou $NR_4R_5$, $S(O)_hR_6$, $(C{=}O)R_7$,

$R_4$, $R_5$ identiques ou différents sont hydrogène, $C_1$-$C_4$ alkyle ou $C_6$-$C_{10}$ aryle, $R_6$ est $C_1$-$C_4$ alkyle,

$R_7$ est $C_1$-$C_4$ alkyle, $C_1$-$C_4$ alkoxy, $C_1$-$C_4$ haloalkoxy, $NR_9R_{10}$,

$R_9$, $R_{10}$ identiques ou différents sont hydrogène ou $C_1$-$C_4$ alkyle,

k = 0, 1, 2, 3, 4, 5,

g = 0, 1, 2, 3, 4,

h = 0, 1, 2,

n'= 0, 1.

**2.** Composé selon la revendication 1, caractérisé en ce que n = 2.

**3.** Composé selon la revendication 1, caractérisé en ce que X = 0.

**4.** Composé selon la revendication 1, caractérisé en ce que m inférieur ou égal à 2.

**5.** Composé selon la revendication 1, caractérisé en ce que p inférieur ou égal à 2.

**6.** Composé selon la revendication 1, caractérisé en ce que k inférieur ou égal à 2.

**7.** Composé selon la revendication 1, caractérisé en ce que g inférieur ou égal à 1.

**8.** Composé selon la revendication 1, caractérisé en ce que $R_1$ est halogène, nitro, trifluorométhyle, méthoxy, méthyle.

**9.** Composé selon la revendication 1, caractérisé en ce que Z est hydrogène.

**10.** Utilisation des composés selon l'une des revendications 1 à 9 à titre d'herbicide notamment sous la forme d'une composition herbicide comportant en outre un support inerte.

**11.** Composition herbicide comportant 0,05 à 95 % en poids d'une matière active selon l'une des revendications 1 à 9 en combinaison avec les supports solides ou liquides acceptables en agriculture et les agents tensio actif acceptables en agriculture.

**12.** Procédé de desherbage (notamment de zones de culture dicotylédone ou de maïs) qui consiste à appliquer aux plantes devant être détruites une quantité efficace d'un composé de formule (I) selon la revendication 1 de préférence en pré émergence.

**13.** Procédé de préparation des composés de formule (I) selon la revendication 1, caractérisé en ce qu'ils sont obtenus:

a)dans le cas où n=2 et A est A1, par mise en contact d'un composé de formule:

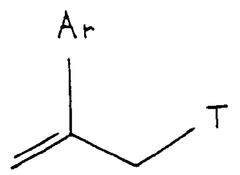

II

dans laquelle Ar, $R_1$ et m ou p ont la même signification que dans la revendication 1, T est un atome de chlore ou de brome, avec un composé de formule :

$MSO_2\,(CH_2)_f\text{-}B$ III

f, B ayant la même définition que celle indiquée dans la revendication 1, M étant un atome de métal alcalin ou alcalino-terreux,

b) dans le cas où A est A1 et n =0, 1, 2 par action du sel alcalin d'un thiolate d'aryle ou d'alkyle de formule :

$M' - S - (CH_2)_f\,B$ VII

dans laquelle M' est un atome de métal alcalin ou alcalinoterreux notamment Na ou K, f et B ayant la même définition que dans la revendication 1 sur un composé de formule (II) où T est halogène précédemment décrit en a) dans un solvant inerte et oxydation éventuelle du sulfur obtenu (n = 0) en sulfoxyde (n = 1) ou sulfone (n = 2),

c) dans le cas où n=2 et A est A1 par réaction d'un composé de formule:

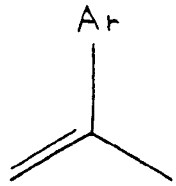

IV

dans laquelle Ar est Ar-1 (noyau phényle), $R_1$ et m ayant la même signification que dans la revendication 1, sur le composé de formule (III) précédemment décrit en a) avec un équivalent d'iode,
d) dans le cas où A est A1, n = 1, 2, par mise en contact de l'anion d'un aryl ou alkylsulfonométhyl phosphonate de dialkyle ou aryle de formule :

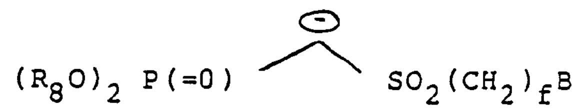

VIII

ou de l'anion d'un aryl ou alkylsulfinométhyl phosphonate de dialkyle ou aryle de formule :

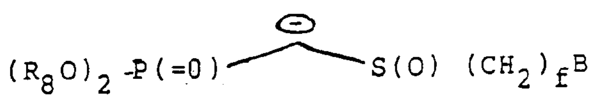

IX

formule dans lesquelles $R_8$ est un groupement $C_1$-$C_4$ alkyle ou $C_6$-$C_{10}$ aryle, f et B ayant la même définition que celle indiquée dans la revendication 1, sur une acétylpyridine ou acétophénone de formule :

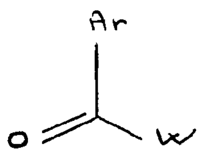

VI

dans laquelle Ar, $R_1$ et m ou p ont la même signification que dans la revendication 1 et W est méthyle, pour fournir les composés de formule X :

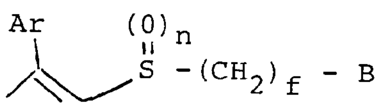

X

dans laquelle Ar, n, f et B ont la même signification que dans la revendication 1, ledit composé de formule X étant ensuite soumis à une étape d'isomérisation par 2 ou plus équivalents de base,
e) dans le cas où A est A1 et n = 2, dans une première étape, par mise en contact de l'anion ou dianion d'une méthylaryl ou alkylsulfone de formule :

$CH_3\ SO_2\ (CH_2)_f\text{-}B$ IIIB

f, B, ayant la même définition que celle indiquée dans la revendication 1, sur une acétophénone ou une acétylpyridine de formule (VI) dans laquelle W est méthyle et Ar, $R_1$ et m ou p ont la même définition que dans la revendication 1 pour fournir les composés de formule :

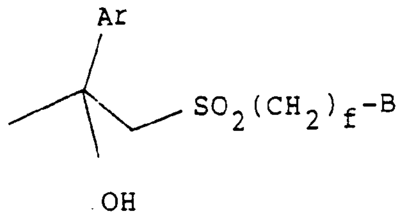

XI

dans laquelle Ar, $R_1$, m ou p, f et B ont la même signification que dans la revendication 1, puis dans une seconde étape par déshydratation du composé de formule XI,
f)dans le cas où A est $A_3$, Y est Cl ou Br , Z est H, par halogénation en milieu aqueux des composés de formule (I) où A est $A_1$
g)dans le cas où A est $A_3$, Y est Cl, Br, I, Z est H, par substitution nucléophile des composés de formule (I) dans laquelle A est $A_3$, Y est $OSO_2R_2$, Cl, Br, Z est H, par le sel alcalin (sodium, potassium, lithium) d'halogénure (Y = Cl, Br, I)
h)dans le cas où A est $A_3$, Y est Cl ou Br, Z est H et n = 2, par mise en contact de l'anion d'une méthylaryl ou alkylsulfone de formule (IIIB), f et B ayant la même définition que celle indiquée dans la revendication 1, sur une ω-halogénoacétophénone ou halogéno acétylpyridine de formule :

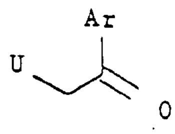

XII

dans laquelle U est un atome de chlore ou de brome, Ar, $R_1$, m ou p ayant la même définition que celle indiquée dans la revendication 1,
i)dans le cas où A est $A_3$, Y est Br ou Cl, Z est H, par ouverture des composés de formule (I) dans laquelle A est $A_2$, X est O, par action d'un réactif donneur d'halogénure Y, Y étant l'atome de chlore ou de brome,
j)dans le cas où A est $A_3$, Y est Br ou Cl, Z est ester, par halogénation par la N-halogéno succinimide, le N-halogéno acétamide, des composés précédemment obtenus de formule (I) où A est $A_1$ en présence d'un acide $R_{11}CO_2H$, $R_{11}$ ayant la même définition que celle indiquée dans la revendication 1
dans le cas où A est $A_3$, Y est I, Z est ester, par substitution nucléophile des composés de formule (I) où A est $A_3$, Y est Br, Z est ester suivant la méthode décrite dans la méthode g.
k)dans le cas où A est $A_3$, Y est $OSO_2R_2$, Z est H, par action de l'halogénure $R_2SO_2$-Hal (Hal étant de préférence l'atome de chlore) sur le composé diol de formule :

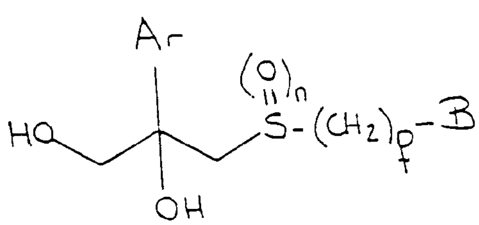

XIII

en présence d'une base azotée comme la pyridine, la triéthylamine,
l)dans le cas où A est $A_2$, X est 0, par cyclisation d'un composé de formule (I) où A est $A_3$ et Y est Cl, Br, I, Z est H, en présence de sel d'argent $Ag_2O$,
dans le cas où n = 0 ou 1 ces mêmes composés de formule (I) dans laquelle A est $A_2$ peuvent également être préparés par cyclisation en milieu basique (NaOH, KOH, $K_2CO_3$ et autres) des composés de formule (I) où A est $A_3$, Y est Cl, Br, I, $OSO_2R_2$, Z est H,
dans le cas où A est ($A_2$) et n =2 ou 1 par oxydation des composés de formule (I) où A est ($A_2$) et n = 1 ou 0,
m)dans le cas où A est $A_2$, n = 2, X = 0, par oxydation d'un composé de formule (I) où A est $A_1$, n = 2,

par des agents d'oxydation,
n)dans le cas où A est $A_2$ et X = O, par déshydratation des composés de formule (XIII), par des agents de déshydratation,
o)dans le cas où A est $A_2$ et X est S, par réaction des composés de formule (I) où A est $A_2$ et X est 0 avec un sel alcalin (généralement potassium) du thiocyanate

**14.** Composés de formules X, XI et XIII selon la revendication 13 utiles, notamment pour la mise en oeuvre du procédé selon la revendication 13.

**Revendications pour les Etats contractants suivants : ES, GR**

**1.** Composition herbicide comportant un composés de formule :

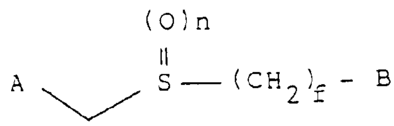

I

dans laquelle :
n = 0, 1, 2
f = 0, 1
A est choisi parmi les groupes

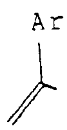 ($A_1$) 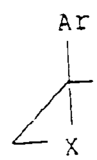 ($A_2$) 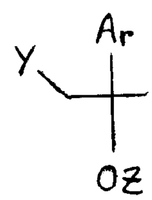 ($A_3$)

dans lesquels :
Ar est choisi parmi les groupes

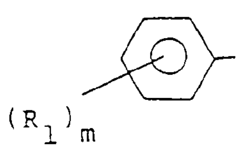
Ar-1

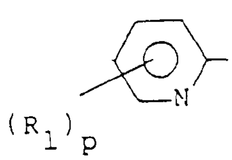
Ar-2

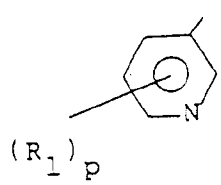
Ar-3

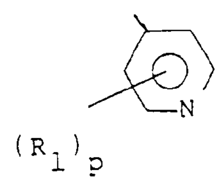
Ar-4

X étant un atome d'oxygène ou de soufre,
$R_1$ étant un atome d'halogène (notamment Cl ou Br ou F), un groupe $C_1$-$C_4$ alkyle, $C_1$-$C_4$ alkoxy, $C_1$-$C_4$ haloalkyle, $C_1$-$C_4$ haloalkoxy, nitro, cyano, $C_6$-$C_{10}$ aryle (notamment phényle ou naphtyle), $C_7$-$C_{11}$ aralkyle (notamment benzyle), $C_6$-$C_{10}$ aryloxy (notamment phénoxy ou napthoxy) éventuellement substitué par 1 ou 2 atomes d'halogène, $C_7$-$C_{11}$
aralkyloxy (notamment benzyloxy) éventuellement substitué par 1 ou 2 atomes d'halogène,
m = 0, 1, 2, 3, 4, 5
p = 0, 1, 2, 3, 4
les différents radicaux $R_1$ étant identiques ou différents lorsque m ou p est supérieur ou égal à 2.
Y est un atome de chlore ou de brome ou d'iode ou un groupement $OSO_2R_2$,
$R_2$ étant un groupement $C_1$-$C_6$ alkyle, $C_6$-$C_{10}$ aryle, $C_7$-$C_{11}$ aralkyle les dits groupements étant éventuellement substitués par 1 à 3 halogènes ou groupements $C_1$-$C_4$ alkyle, $C_1$-$C_4$ alkoxy, $C_1$-$C_4$ haloalkyle, $C_1$-$C_4$ haloalkoxy, nitro,
Z étant un atome d'hydrogène ou un groupe (C=0)$R_{11}$, $R_{11}$ est hydrogène, $C_1$-$C_4$ alkyle, $C_1$-$C_4$ haloalkyle,

B est choisi parmi les groupes $C_1$-$C_{10}$ alkyle,
$C_3$-$C_{10}$ cycloalkyle, ces groupes étant éventuellement substitués par 1 à 6 atomes d'halogène ou choisi parmi les groupes

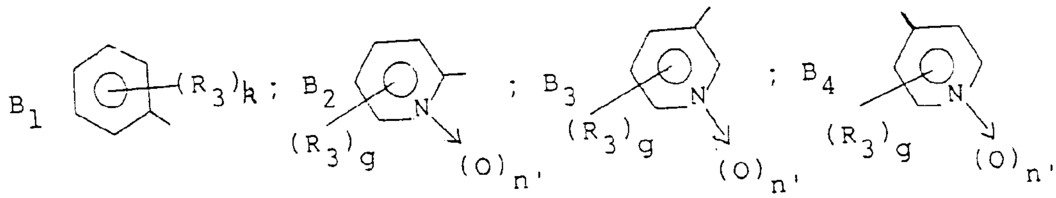

$R_3$ ayant l'une des significations indiquées pour $R_1$ ou $NR_4R_5$, $S(0)_hR_6$, $(C{=}0)R_7$,
$R_4$, $R_5$ identiques ou différents sont hydrogène,
$C_1$-$C_4$ alkyle ou $C_6$-$C_{10}$ aryle, $R_6$ est $C_1$-$C_4$ alkyle,
$R_7$ est $C_1$-$C_4$ alkyle, $C_1$-$C_4$ alkoxy, $C_1$-$C_4$ haloalkoxy, $NR_9R_{10}$,
$R_9$, $R_{10}$ identiques ou différents sont hydrogène ou $C_1$-$C_4$ alkyle,
k = 0, 1, 2, 3, 4, 5,
g = 0, 1, 2, 3, 4,
h = 0, 1, 2,
n' = 0, 1.
avec un support inerte.

2. Composition selon la revendication 1, caractérisé en ce que n = 2.

3. Composition selon la revendication 1, caractérisé en ce que X = 0.

4. Composition selon la revendication 1, caractérisé en ce que m inférieur ou égal à 2.

5. Composition selon la revendication 1, caractérisé en ce que p inférieur ou égal à 2.

6. Composition selon la revendication 1, caractérisé en ce que k inférieur ou égal à 2.

7. Composition selon la revendication 1, caractérisé en ce que g inférieur ou égal à 1.

8. Composition selon la revendication 1, caractérisé en ce que $R_1$ est halogène, nitro, trifluorométhyle, méthoxy, méthyle.

9. Composition selon la revendication 1, caractérisé en ce que Z est hydrogène.

10. Utilisation des composés selon l'une des revendications 1 à 9 à titre d'herbicide.

11. Composition herbicide comportant 0,05 à 95 % en poids d'une matière active selon l'une des revendications 1 à 9 en combinaison avec les supports solides ou liquides acceptables en agriculture et les agents tensio actif acceptables en agriculture.

12. Procédé de desherbage (notamment de zones de culture dicotylédone ou de maïs) qui consiste à appliquer aux plantes devant être détruites une quantité efficace d'un composé de formule (I) selon la revendication 1 de préférence en pré émergence.

13. Procédé de préparation des composés de formule (I) selon la revendication 1, caractérisé en ce qu'ils sont obtenus:

a)dans le cas où n=2 et A est Al, par mise en contact d'un composé de formule:

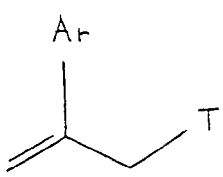

II

dans laquelle Ar, $R_1$ et m ou p ont la même signification que dans la revendication 1, T est un atome de chlore ou de brome, avec un composé de formule :

$MSO_2\ (CH_2)_f$ - B III

f, B ayant la même définition que celle indiquée dans !a revendication 1, M étant un atome de métal alcalin ou alcalino-terreux,

b) dans le cas où A est A1 et n =0, 1, 2 par action du sel alcalin d'un thiolate d'aryle ou d'alkyle de formule :

M' - S - $(CH_2)_f$ B VII

dans laquelle M' est un atome de métal alcalin ou alcalinoterreux notamment Na ou K, f et B ayant la même définition que dans la revendication 1 sur un composé de formule (II) où T est halogène précédemment décrit en a) dans un solvant inerte et oxydation éventuelle du sulfur obtenu (n = 0) en sulfoxyde (n = 1) ou sulfone (n = 2),

c) dans le cas où n=2 et A est A1 par réaction d'un composé de formule:

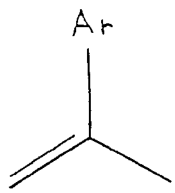

IV

dans laquelle Ar est Ar-1 (noyau phényle), $R_1$ et m ayant la même signification que dans la revendication 1, sur le composé de formule (III) précédemment décrit en a) avec un équivalent d'iode,

d) dans le cas où A est A1, n = 1, 2, par mise en contact de l'anion d'un aryl ou alkylsulfonométhyl phosphonate de dialkyle ou aryle de formule :

$(R_8O)_2$ P(=O) $SO_2(CH_2)_fB$ VIII

ou de l'anion d'un aryl ou alkylsulfinométhyl phosphonate de dialkyle ou aryle de formule :

$(R_8O)_2$ P(=O) S(O) $(Ch_2)_fB$ IX

formule dans lesquelles $R_8$ est un groupement $C_1$-$C_4$ alkyle ou $C_6$-$C_{10}$ aryle, f et B ayant la même définition que celle indiquée dans la revendication 1, sur une acétylpyridine ou acétophénone de formule :

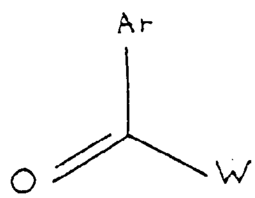

VI

dans laquelle Ar, $R_1$ et m ou p ont la même signification que dans la revendication 1 et W est méthyle, et isomérisation du composé vinylique obtenu par 2 ou plus équivalents de base,

e) dans le cas où A est A1 et n = 2, dans une première étape, par mise en contact de l'anion ou dianion d'une méthylaryl ou alkylsulfone de formule :

$CH_3\ SO_2\ (CH_2)_f$-B IIIB

f, B, ayant la même définition que celle indiquée dans la revendication 1, sur une acétophénone ou une acétylpyridine de formule (VI) dans laquelle W est méthyle et Ar, $R_1$ et m ou p ont la même définition que dans la revendication 1 pour fournir les composés de formule :

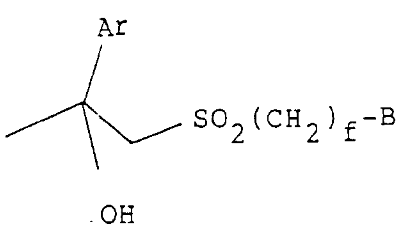

XI

dans laquelle Ar, $R_1$, m ou p, f et B ont la même signification que dans la revendication 1, puis dans une seconde étape par déshydratation du composé de formule XI,

f)dans le cas où A est $A_3$, Y est Cl ou Br , Z est H, par halogénation en milieu aqueux des composés de formule (I) où A est $A_1$

g)dans le cas où A est $A_3$, Y est Cl, Br, I, Z est H, par substitution nucléophile des composés de formule (I) dans laquelle A est $A_3$, Y est $OSO_2R_2$, Cl, Br, Z est H, par le sel alcalin (sodium, potassium, lithium) d'haloaénure (Y = Cl, Br, I)

h)dans le cas où A est $A_3$, Y est Cl ou Br, Z est H et n = 2, par mise en contact de l'anion d'une méthylaryl ou alkylsulfone de formule (IIIB), f et B ayant la même définition que celle indiquée dans la revendication 1, sur une ω-halogénoacétophénone ou halogéno acétylpyridine de formule :

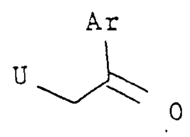

XII

dans laquelle U est un atome de chlore ou de brome, Ar, $R_1$, m ou p ayant la même définition que celle indiquée dans la revendication 1,

i)dans le cas où A est $A_3$, Y est Br ou Cl, Z est H, par ouverture des composés de formule (I) dans laquelle A est $A_2$, X est O, par action d'un réactif donneur d'halogénure $Y^-$, Y étant l'atome de chlore ou de brome,

j)dans le cas où A est $A_3$, Y est Br ou Cl, Z est ester, par halogénation par la N-halogéno succinimide, le N-halogéno acétamide, des composés précédemment obtenus de formule (I) où A est $A_1$ en présence d'un acide $R_{11}CO_2H$, $R_{11}$ ayant la même définition que celle indiquée dans la revendication 1

dans le cas où A est $A_3$, Y est I, Z est ester, par substitution nucléophile des composés de formule (I) où A est $A_3$, Y est Br, Z est ester suivant la méthode décrite dans la méthode g.

k)dans le cas où A est $A_3$, Y est $OSO_2R_2$, Z est H, par action de l'halogénure $R_2SO_2$-Hal (Hal étant de préférence l'atome de chlore) sur le composé diol de formule :

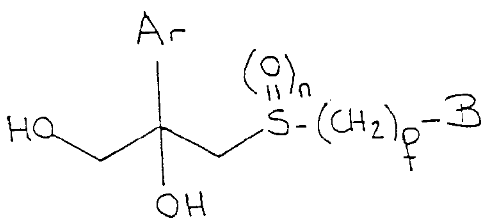

XIII

en présence d'une base azotée comme la pyridine, la triéthylamine,

l)dans le cas où A est $A_2$, X est O, par cyclisation d'un composé de formule (I) où A est $A_3$ et Y est Cl, Br, I, Z est H, en présence de sel d'argent $Ag_2O$,

dans le cas où n = 0 ou 1 ces mêmes composés de formule (I) dans laquelle A est $A_2$ peuvent également être préparés par cyclisation en milieu basique (NaOH, KOH, $K_2CO_3$ et autres) des composés de formule (I) où A est $A_3$, Y est Cl, Br, I, $OSO_2R_2$, Z est H,

dans le cas où A est ($A_2$) et n =2 ou 1 par oxydation des composés de formule (I) où A est ($A_2$) et n = 1 ou 0,

m)dans le cas où A est $A_2$, n = 2, X = 0, par oxydation d'un composé de formule (I) où A est $A_1$, n = 2, par des agents d'oxydation,

n)dans le cas où A est $A_2$ et X = O, par déshydratation des composés de formule (XIII), par des agents de déshydratation,

o)dans le cas où A est $A_2$ et X est S, par réaction des composés de formule (I) où A est $A_2$ et X est O avec un sel alcalin (généralement potassium) du thiocyanate

## Patentansprüche

## Patentansprüche für folgende Vertragsstaaten : AT, BE, CH, DE, FR, GB, IT, LI, LU, NL, SE

1. Verbindungen der Formel

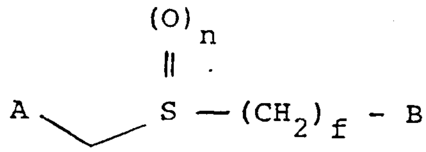

I

in der:

n = 0, 1, 2

f = 0,1

A ausgewählt wird aus den Resten

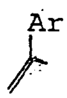 $(A_1)$ 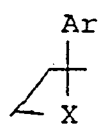 $(A_2)$ 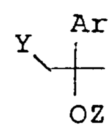 $(A_3)$

in denen:

Ar ausgewählt wird aus den Resten

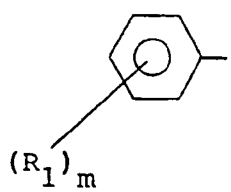 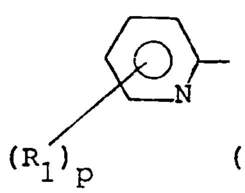 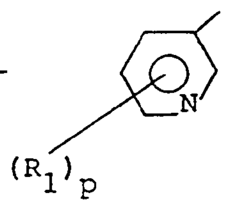 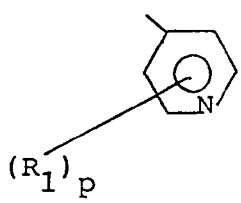

Ar-1 Ar-2 Ar-3 Ar-4

X ein Sauerstoff- oder Schwefelatom ist

$R_1$ ein Halogenatom (insbesondere Cl oder Br oder F),

eine $C_1$-$C_4$ Alkylgruppe, $C_1$-$C_4$-Alkoxygruppe, $C_1$-$C_4$-Haloalkylgruppe, $C_1$-$C_4$-Haloalkoxygruppe, Nitrogruppe,

Cyanogruppe, $C_6$-$C_{10}$-Arylgruppe (insbesondere Phenyl oder Naphthyl) $C_7$-$C_{11}$-Aralkylgruppe (insbesondere Benzyl, $C_6$-$C_{10}$ Aryloxygruppe (insbesondere Phenoxy oder Naphthoxy), gegebenenfalls substituiert mit 1 oder 2 Halogenatomen, $C_7$-$C_{11}$ Aralkyloxygruppe (insbesondere Benzyloxy) gegebenenfalls substituiert mit 1 oder 2 Halogenatomen, ist,

m = 0, 1, 2, 3, 4, 5

p = 0, 1, 2, 3, 4

die verschiedenen Reste $R_1$ identisch oder verschieden sind, wenn m oder p größer oder gleich 2 ist,

Y ein Chlor- oder Brom- oder Jodatom oder eine Gruppierung $OSO_2R_2$ ist, wobei $R_2$ ein Rest $C_1$-$C_6$ Alkyl, $C_6$-$C_{10}$ Aryl, $C_7$-$C_{11}$ Aralkyl ist, wobei diese Reste gegebenenfalls mit 1 bis 3 Halogen oder $C_1$-$C_4$ Alkyl, $C_1$-$C_4$ Alkoxy, $C_1$-$C_4$ Haloalkyl,

$C_1$-$C_4$ Haloalkoxy oder Nitrogruppen substituiert sind,

Z ein Wasserstoffatom oder ein Rest $(C{=}O)R_{11}$ ist, wobei $R_{11}$ Wasserstoff, $C_1$-$C_4$ Alkyl, oder $C_1$-$C_4$ Haloalkyl ist,

B ausgewählt wird aus den Resten $C_1$-$C_{10}$ Alkyl, $C_3$-$C_{10}$ Alkyl, $C_3$-$C_{10}$ Cycloalkyl, wobei diese Reste gegebenenfalls mit 1 bis 6 Halogenatomen substituiert sind oder aus den Resten

$B_1$ 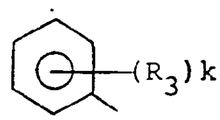 ; $B_2$ 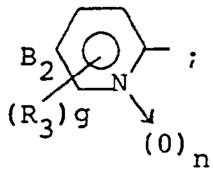 ; $B_3$ 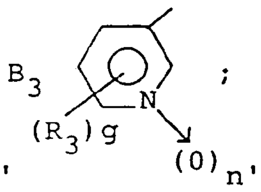 ; $B_4$ 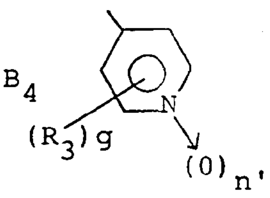

ausgewählt wird,
wobei $R_3$ eine der für $R_1$ angegebenen Bedeutungen hat oder $NR_4R_5$, $S(O)_hR_6$, $(C{=}O)R_7$ ist, wobei $R_4$ und $R_5$ identisch oder unterschiedlich sind und Wasserstoff, $C_1$-$C_4$ Alkyl oder $C_6$-$C_{10}$ Aryl darstellen, $R_5$ $C_1$-$C_4$ Alkyl ist, $R_7$ $C_1$-$C_4$ Alkyl, $C_1$-$C_4$ Alkoxy, $C_1$-$C_4$ Haloalkoxy ist oder $NR_9R_{10}$, wobei $R_9$ und $R_{10}$ identisch oder verschieden sind und Wasserstoff oder $C_l$-$C_4$ Alkyl darstellen,
k = 0, 1, 2, 3, 4, 5
g = 0, 1, 2, 3, 4
h = 0, 1, 2
n' = 0, 1
ist.

2. Verbindung nach Anspruch 1, gekennzeichnet dadurch, daß n = 2 ist.

3. Verbindung nach Anspruch 1, gekennzeichnet dadurch, daß X = 0 ist.

4. Verbindung nach Anspruch 1, gekennzeichnet dadurch, daß m kleiner oder gleich 2 ist.

5. Verbindung nach Anspruch 1, gekennzeichnet dadurch, daß p kleiner oder gleich 2 ist.

6. Verbindung nach Anspruch 1, gekennzeichnet dadurch, daß k kleiner oder gleich 2 ist.

7. Verbindung nach Anspruch 1, gekennzeichnet dadurch, daß g kleiner oder gleich 1 ist.

8. Verbindung nach Anspruch 1, gekennzeichnet dadurch, daß $R_1$ Halogen, Nitro, Trifluormethyl, Methoxy oder Methyl ist.

9. Verbindung nach Anspruch 1, gekennzeichnet dadurch, daß Z Wasserstoff ist.

10. Verwendung von Verbindungen gemäß einem der Ansprüche 1 bis 9 als Herbizide, insbesondere in Form einer herbiziden Zusammensetzung enthaltend außerdem einen inerten Träger.

11. Herbizide Zusammensetzung enthaltend 0,05 bis 95 Gew.% eines Wirkstoffs gemäß einem der Ansprüche 1 bis 9 in Verbindung mit festen oder flüssigen in der Landwirtschaft verträglichen Trägern und in der Landwirtschaft verträglichen oberflächenaktiven Agenzien.

12. Verfahren zur Entfernung von Unkraut (insbesondere in Gebieten mit Kulturen von Dicotyledonen oder Mais), das darin besteht auf die Pflanzen, die zerstört werden sollen, eine wirksame Menge einer Verbindung der Formel (I) gemäß Anspruch 1 vorzugsweise vor dem Auflauf anzuwenden.

13. Verfahren zur Herstellung von Verbindungen der Formel (I) gemäß Anspruch 1, gekennzeichnet dadurch, daß sie erhalten werden:
a) in dem Fall in dem n=2 und A gleich $A_1$ ist, durch in Kontakt bringen einer Verbindung der Formel

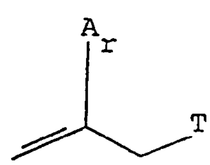 II

in der Ar, $R_1$ und m oder p die gleiche Bedeutung haben wie in Anspruch 1, T ein Chlor- oder Bromatom ist, mit einer Verbindung der Formel:

$MSO_2\,(CH_2)_f$ - B III

worin f und B die gleiche Bedeutung haben wie oben in Anspruch 1 angegeben, worin M ein Alkalimetall oder Erdalkalimetallatom ist,

b) in dem Fall in dem A $A_1$ ist und n = 0, 1 oder 2 ist, durch Einwirkung eines Alkalisalzes eines Aryl- oder Alkylthiolats der Formel:

M' - S - $(CH_2)_f$ B VII

in der M' ein Alkali- oder Erdalkalimetallatom ist, insbesondere Na oder K, in der f und B die gleiche Bedeutung haben wie in Anspruch 1, auf eine Verbindung der Formel (II) in der T Halogen ist wie unter a) beschrieben, in einem inerten Lösungsmittel und gegebenenfalls Oxidation des erhaltenen Schwefels (n = 0) in das Sulfoxid (n = 1) oder Sulfon (n = 2),

c) in dem Fall in dem n=2 ist und A gleich $A_1$ ist durch Reaktion einer Verbindung der Formel

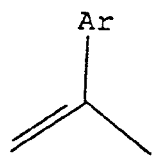
IV

in der Ar gleich Ar-1 ist (Phenylkern) $R_1$ und m die gleiche Bedeutung haben wie in Anspruch 1, mit einer Verbindung der Formel (III) wie sie oben unter a) beschrieben ist mit einem Äquivalent Jod

d) in dem Fall in dem A $A_1$ ist, n=1 oder 2 ist, durch in Kontakt bringen des Anions eines (Dialkyl) oder (Aryl) Aryl- oder Alkylsulfonmethylphosphonats der Formel:

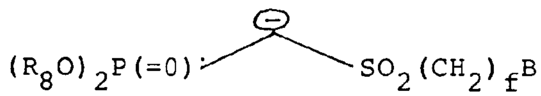
VIII

oder eines (Dialkyl) oder (Aryl) Aryl- oder Alkylsulfinomethylphosphonats der Formel

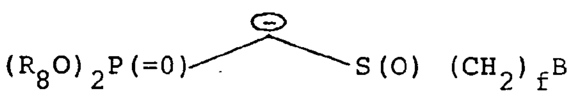
IX

wobei in den Formeln $R_8$ ein $C_1$-$C_4$ Alkyl oder $C_6$-$C_{10}$ Arylrest ist, f und B die gleiche Bedeutung haben wie oben bei Anspruch 1 angegeben, mit einem Acetylpyridin oder Acetophenon der Formel:

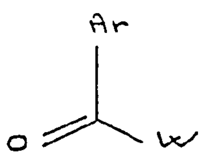
VI

in der Ar, $R_1$ und m oder p die gleiche Bedeutung haben wie in Anspruch 1 und W Methyl ist, um Verbindungen der Formel X zu liefern:

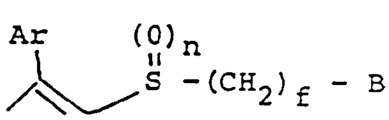
X

in der Ar, n, f und B die gleiche Bedeutung wie im Anspruch 1 haben, worauf die Verbindung der Formel X mit zwei oder mehr Äquivalenten einer Base einer Isomerisierung unterworfen wird.

e) in dem Fall in dem A $A_1$ ist und n = 2 ist, in einem ersten Schritt, durch in Kontakt bringen des Anions oder Dianions eines Methylaryl- oder Alkylsulfons der Formel:

$CH_3\,SO_2\,(CH_2)_f$-B IIIB

in der f und B die gleiche Bedeutung haben wie in Anspruch 1 angegeben, mit einem Acetophenon oder

einem Acetylpyridin der Formel (VI) in der W Methyl ist und Ar, $R_1$ und m oder p die gleiche Bedeutung haben wie in Anspruch 1, wobei die Verbindungen der Formel:

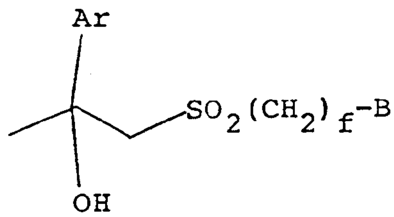 XI

erhalten werden in der Ar, $R_1$ m oder p, f und B die gleiche Bedeutung haben wie in Anspruch 1, und dann in einem zweiten Schritt durch Dehydratisierung der Verbindung der Formel XI,
f) in dem Fall in dem A $A_3$ ist, Y Cl oder Br ist, Z gleich H ist, durch Halogenierung in wässrigem Milieu von Verbindungen der Formel (I) in denen A $A_1$ ist
g) in dem Fall in dem A gleich $A_3$ ist, Y, Cl, Br oder J ist, Z H ist, durch nukleophile Substitution von Verbindungen der Formel (I) in der A $A_3$ ist, Y $OSO_2R_2$, Cl oder Br ist, Z H ist, durch das Alkalisalz (Natrium, Kalium, Lithium) des Halogenids (Y = Cl, Br, J)
h) in dem Fall in dem A $A_3$ ist, Y Cl oder Br ist, Z H ist und n = 2 ist, durch in Kontakt bringen des Anions eines Methylaryl- oder Alkylsulfons der Formel (IIIB), in der f und B die gleiche Bedeutung haben wie in Anspruch 1 angegeben, mit einem -Halogenacetophenon oder Halogenacetylpyridin der Formel

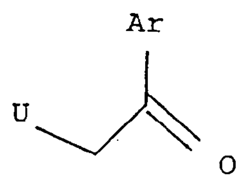 XII

in der U ein Chlor- oder Bromatom ist, Ar, $R_1$, m oder p die gleiche Bedeutung haben wie in Anspruch 1 angegeben,
i) in dem Fall in dem A $A_3$ ist, Y gleich Br oder Cl ist, Z H ist, durch Öffnung von Verbindungen der Formel (I) in der A $A_2$ ist, X O ist, durch Einwirkung eines Halogenid-(Y)-Donor-Reagenzes, worin Y ein Chlor- oder Bromatom ist,
j) in dem Fall in dem A $A_3$ ist, Y Br oder Cl ist,
Z ein Ester ist, durch Halogenierung durch das N-Halogensuccinimid oder N-Halogenacetamid, der vorher erhaltenen Verbindungen der Formel (I) bei denen A $A_1$ ist in Gegenwart einer Säure $R_{11}CO_2H$, wobei $R_{11}$ die gleiche Bedeutung hat wie in Anspruch 1 angegeben,
in dem Fall in dem A $A_3$ ist, Y J ist, Z ein Ester ist, durch nukleophile Substitution von Verbindungen der Formel (I) bei denen A $A_3$ ist, Y Br ist, Z ein Ester ist, nach der in Methode g beschriebenen Methode,
k) in dem Fall in dem A $A_3$ ist, Y $OSO_2R_2$ ist, Z H ist, durch Einwirkung eines Halogenids $R_2SO_2$-Hal (Hal ist vorzugsweise Chlor) auf eine Diolverbindung der Formel

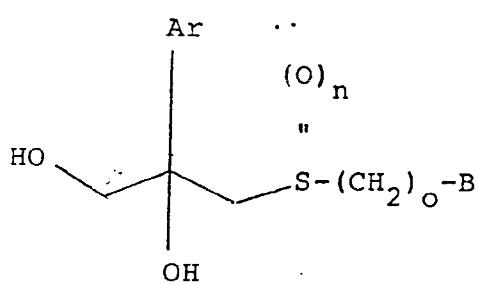 XIII

in Gegenwart einer Stickstoffbase wie Pyridin oder Triethylamin,
l) in dem Fall in dem A $A_2$ ist, X gleich 0 ist, durch Cyclysierung einer Verbindung der Formel (I) in der A $A_3$ ist Y Cl, Br oder J ist und Z H ist, in Gegenwart eines Silbersalzes $Ag_2O$, in dem Fall in dem n = 0 oder 1 ist können die gleichen Verbindungen der Formel (I) in denen A $A_2$ ist auch durch Cyclysierung

in basischem Milieu (NaOH, KOH, $K_2CO_3$ und andere) aus Verbindungen der Formel I) hergestellt werden, in denen A $A_3$ ist, Y Cl, Br, J, $OSO_2R_2$ ist, Z gleich H ist,
in dem Fall in dem A ($A_2$) ist und n = 2 oder 1 ist, durch Oxidation von Verbindungen der Formel (I) in denen A ($A_2$) ist und n = 1 oder 0 ist,
m) in dem Fall in dem A $A_2$ ist, n = 2, X = 0 ist, durch Oxidation einer Verbindung der Formel (I) in der A $A_1$ ist, n = 2 ist, durch Oxidationsmittel,
n) in dem Fall in dem A $A_2$ ist und X = 0 ist, durch Dehydratisierung von Verbindungen der Formel (XIII) mit Hilfe von Dehydratisierungsagenzien,
o) in dem Fall in dem A $A_2$ ist oder X S ist, durch Reaktion von Verbindungen der Formel (I) in denen A $A_2$ ist und X O ist mit einem Alkalisalz (im allgemeinen Kalium) eines Thiocyanats.

**14.** Verbindungen der Formeln X, XI und XIII gemäß Anspruch 13, die insbesondere für die Durchführung des Verfahrens gemäß Anspruch 13 verwendbar sind.

**Patentansprüche für folgende Vertragsstaaten : ES, GR**

**1.** Herbizide Zusammensetzung enthaltend eine Verbindung der Formel

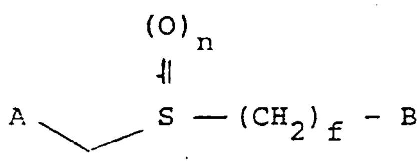 I

in der:
n = 0, 1, 2
f = 0,1
A ausgewählt wird aus den Resten

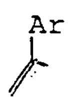 ($A_1$) 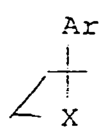 ($A_2$) 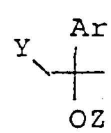 ($A_3$)

in denen:
Ar ausgewählt wird aus den Resten

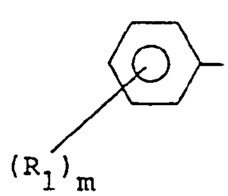 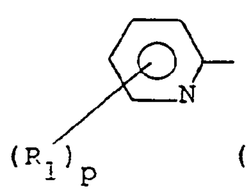 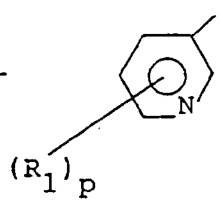 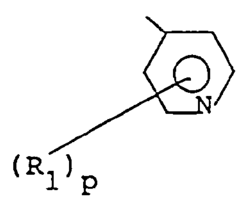

Ar-1 Ar-2 Ar-3 Ar-4

X ein Sauerstoff- oder Schwefelatom ist
$R_1$ ein Halogenatom (insbesondere Cl oder Br oder F), eine $C_1$-$C_4$ Alkylgruppe, $C_1$-$C_4$-Alkoxygruppe, $C_1$-$C_4$-Haloalkylgruppe, $C_1$-$C_4$-Haloalkoxygruppe, Nitrogruppe, Cyanogruppe, $C_6$-$C_{10}$-Arylgruppe (insbesondere Phenyl oder Naphthyl) $C_7$-$C_{11}$-Aralkylgruppe (insbesondere Benzyl, $C_6$-$C_{10}$ Aryloxygruppe (insbesondere Phenoxy oder Naphthoxy), gegebenenfalls substituiert mit 1 oder 2 Halogenatomen, $C_7$-$C_{11}$ Aralkyloxygruppe (insbesondere Benzyloxy) gegebenenfalls substituiert mit 1 oder 2 Halogenatomen, ist,
m = 0, 1, 2, 3, 4, 5
p = 0, 1, 2, 3, 4
die verschiedenen Reste $R_1$ identisch oder verschieden sind, wenn m oder p größer oder gleich 2 ist,

Y ein Chlor- oder Brom- oder Jodatom oder eine Gruppierung $OSO_2R_2$ ist, wobei $R_2$ ein Rest $C_1$-$C_6$-Alkyl, $C_6$-$C_{10}$ Aryl, $C_7$-$C_{11}$ Aralkyl ist, wobei diese Reste gegebenenfalls mit 1 bis 3 Halogen oder $C_1$-$C_4$ Alkyl, $C_1$-$C_4$ Alkoxy, $C_1$-$C_4$ Haloalkyl, $C_1$-$C_4$ Haloalkoxy oder Nitrogruppen substituiert sind,
Z ein Wasserstoffatom oder ein Rest $(C{=}O)R_{11}$ ist, wobei $R_{11}$ Wasserstoff, $C_1$-$C_4$ Alkyl, oder $C_1$-$C_4$ Haloalkyl ist,
B ausgewählt wird aus den Resten $C_1$-$C_{10}$ Alkyl, $C_3$-$C_{10}$ Alkyl, $C_3$-$C_{10}$ Cycloalkyl, wobei diese Reste gegebenenfalls mit 1 bis 6 Halogenatomen substituiert sind oder aus den Resten

$B_1$ 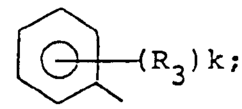 ; $B_2$ 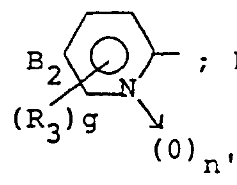 ; $B_3$ 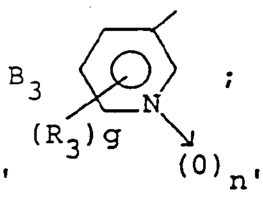 ; $B_4$ 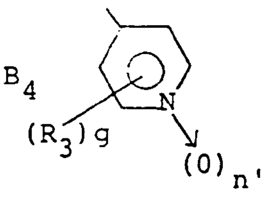

ausgewählt wird,
wobei $R_3$ eine der für $R_1$ angegebenen Bedeutungen hat oder $NR_4R_5$, $S(O)_hR_6$, $(C{=}O)R_7$ ist,
wobei $R_4$ und $R_5$ identisch oder unterschiedlich sind und Wasserstoff, $C_1$-$C_4$ Alkyl oder $C_6$-$C_{10}$ Aryl darstellen, $R_5$ $C_1$-$C_4$ Alkyl ist, $R_7$ $C_1$-$C_4$ Alkyl, $C_l$-$C_4$ Alkoxy, $C_1$-$C_4$ Haloalkoxy ist oder $NR_9R_{10}$, wobei $R_9$ und $R_{10}$ identisch oder verschieden sind und Wasserstoff oder $C_1$-$C_4$ Alkyl darstellen,
k = 0, 1, 2, 3, 4, 5
g = 0, 1, 2, 3, 4
h = 0, 1, 2
n′ = 0, 1
ist, mit einem inerten Träger.

2. Zusammensetzung nach Anspruch 1, gekennzeichnet dadurch, daß n = 2 ist.

3. Zusammensetzung nach Anspruch 1, gekennzeichnet dadurch, daß X = 0 ist.

4. Zusammensetzung nach Anspruch 1, gekennzeichnet dadurch, daß m kleiner oder gleich 2 ist.

5. Zusammensetzung nach Anspruch 1, gekennzeichnet dadurch, daß p kleiner oder gleich 2 ist.

6. Zusammensetzung nach Anspruch 1, gekennzeichnet dadurch, daß k kleiner oder gleich 2 ist.

7. Zusammensetzung nach Anspruch 1, gekennzeichnet dadurch, daß g kleiner oder gleich 1 ist.

8. Zusammensetzung nach Anspruch 1, gekennzeichnet dadurch, daß $R_1$ Halogen, Nitro, Trifluormethyl, Methoxy oder Methyl ist.

9. Zusammensetzung nach Anspruch 1, gekennzeichnet dadurch, daß Z Wasserstoff ist.

10. Verwendung von Verbindungen gemäß einem der Ansprüche 1 bis 9 als Herbizide.

11. Herbizide Zusammensetzung enthaltend 0,05 bis 95 Gew.% eines Wirkstoffs gemäß einem der Ansprüche 1 bis 9 in Verbindung mit festen oder flüssigen in der Landwirtschaft verträglichen Trägern und in der Landwirtschaft verträglichen oberflächenaktiven Agenzien.

12. Verfahren zur Entfernung von Unkraut (insbesondere in Gebieten mit Kulturen von Dicotyledonen oder Mais), das darin besteht auf die Pflanzen, die zerstört werden sollen, eine wirksame Menge einer Zusammensetzung der Formel (I) gemäß Anspruch 1 vorzugsweise vor dem Auflauf anzuwenden.

13. Verfahren zur Herstellung von Verbindungen der Formel (I) gemäß Anspruch 1, gekennzeichnet dadurch, daß sie erhalten werden:
a) in dem Fall in dem n=2 und A gleich $A_1$ ist, durch in Kontakt bringen einer Verbindung der Formel

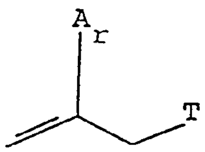

II

in der Ar, $R_1$ und m oder p die gleiche Bedeutung haben wie in Anspruch 1, T ein Chlor- oder Bromatom ist, mit einer Verbindung der Formel:

$MSO_2\ (CH_2)_f$ - B III

worin f und B die gleiche Bedeutung haben wie oben in Anspruch 1 angegeben, worin M ein Alkalimetall oder Erdalkalimetallatom ist,

b) in dem Fall in dem A $A_1$ ist und n = 0, 1 oder 2 ist, durch Einwirkung eines Alkalisalzes eines Aryl- oder Alkylthiolats der Formel:

M' - S - $(CH_2)_f$ B VII

in der M' ein Alkali- oder Erdalkalimetallatom ist, insbesondere Na oder K, in der f und B die gleiche Bedeutung haben wie in Anspruch 1, auf eine Verbindung der Formel (II) in der T halogen ist wie unter a) beschrieben, in einem inerten Lösungsmittel und gegebenenfalls Oxidation des erhaltenen Schwefels (n = 0) in das Sulfoxid (n = 1) oder Sulfon (n = 2),

c) in dem Fall in dem n=2 ist und A gleich $A_1$ ist durch Reaktion einer Verbindung der Formel

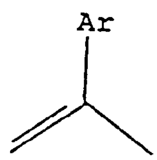

IV

in der Ar gleich Ar-1 ist (Phenylkern) $R_1$ und m die gleiche Bedeutung haben wie in Anspruch 1, mit einer Verbindung der Formel (III) wie sie oben unter a) beschrieben ist mit einem Äquivalent Jod

d) in dem Fall in dem A $A_1$ ist, n=1 oder 2 ist, durch in Kontakt bringen des Anions eines (Dialkyl) oder (Aryl) Aryl- oder Alkylsulfonmethylphosphonats der Formel:

$(R_8O)_2P$ (=0) $SO_2(CH_2)_fB$ VIII

oder eines (Dialkyl) oder (Aryl) Aryl- oder Alkylsulfinomethylphosphonats der Formel

$(R_8O)_2P$ (=0) S(O) $(CH_2)_fB$ IX

wobei in den Formeln $R_8$ ein $C_1$-$C_4$ Alkyl oder $C_6$-$C_{10}$ Arylrest ist, f und B die gleiche Bedeutung haben wie oben bei Anspruch 1 angegeben, mit einem Acetylpyridin oder Acetophenon der Formel:

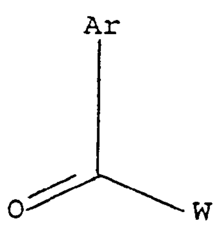

VI

in der Ar, $R_1$ und m oder p die gleiche Bedeutung haben wie in Anspruch 1 und W Methyl ist, und Isomerisierung der erhaltenen Vinylverbindung mit zwei oder mehr Äquivalenten einer Base,

e) in dem Fall in dem A $A_1$ ist und n = 2 ist, in einem ersten Schritt, durch in Kontakt bringen des Anions oder Dianions eines Methylaryl- oder Alkylsulfons der Formel:

$CH_3\ SO_2\ (CH_2)_f$-B IIIB

in der f und B die gleiche Bedeutung haben wie in Anspruch 1 angegeben, mit einem Acetophenon oder einem Acetylpyridin der Formel (VI) in der W Methyl ist und Ar, $R_1$ und m oder p die gleiche Bedeutung haben wie in Anspruch 1, wobei die Verbindungen der formel:

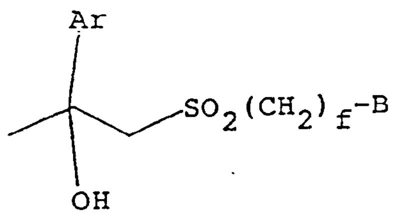

XI

erhalten werden in der Ar, $R_1$ m oder p, f und B die gleiche Bedeutung haben wie in Anspruch 1, und dann in einem zweiten Schritt durch Dehydratisierung der Zusammensetzung der Formel XI,

f) in dem Fall in dem A $A_3$ ist, Y Cl oder Br ist, Z gleich H ist, durch Halogenierung in wässrigem Milieu von Verbindungen der Formel (I) in denen A $A_1$ ist

g) in dem Fall in dem A gleich $A_3$ ist, Y, Cl, Br oder J ist, Z H ist, durch nukleophile Substitution von Verbindungen der Formel (I) in der A $A_3$ ist, Y $OSO_2R_2$, Cl oder Br ist, Z H ist, durch das Alkalisalz (Natrium, Kalium, Lithium) des Halogenids (Y = Cl, Br, J)

h) in dem Fall in dem A $A_3$ ist, Y Cl oder Br ist, Z H ist und n = 2 ist, durch in Kontakt bringen des Anions eines Methylaryl- oder Alkylsulfons der Formel (IIIB), in der f und B die gleiche Bedeutung haben wie in Anspruch 1 angegeben, mit einem -Halogenacetophenon oder Halogenacetylpyridin der Formel

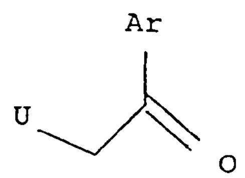

XII

in der U ein Chlor- oder Bromatom ist, Ar, $R_1$, m oder p die gleiche Bedeutung haben wie in Anspruch 1 angegeben,

i) in dem Fall in dem A $A_3$ ist, Y gleich Br oder Cl ist, Z H ist, durch Öffnung von Verbindungen der Formel (I) in der A $A_2$ ist, X O ist, durch Einwirkung eines Halogenid-(Y)-Donor-Reagenzes, worin Y ein Chlor- oder Bromatom ist,

j) in dem Fall in dem A $A_3$ ist, Y Br oder Cl ist, Z ein Ester ist, durch Halogenierung durch das N-Halogensuccinimid oder N-Halogenacetamid, der vorher erhaltenen Verbindungen der Formel (I) bei denen A $A_1$ ist in Gegenwart einer Säure $R_{11}CO_2H$, wobei $R_{11}$ die gleiche Bedeutung hat wie in Anspruch 1 angegeben,

in dem Fall in dem A $A_3$ ist, Y J ist, Z ein Ester ist, durch nukleophile Substitution von Verbindungen der Formel (I) bei denen A $A_3$ ist, Y Br ist, Z ein Ester ist, nach der in Methode g beschriebenen Methode,

k) in dem Fall in dem A $A_3$ ist, Y $OSO_2R_2$ ist, Z H ist, durch Einwirkung eines Halogenids $R_2SO_2$-Hal (Hal ist vorzugsweise Chlor) auf eine Diolverbindung der Formel

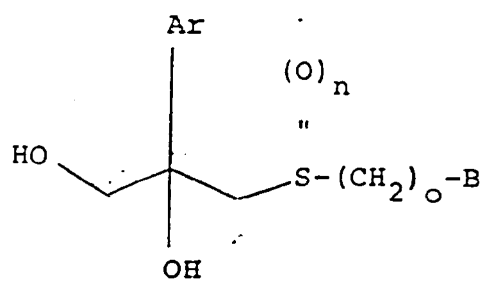

XIII

in Gegenwart einer Stickstoffbase wie Pyridin oder Triethylamin,

l) in dem Fall in dem A $A_2$ ist, X gleich 0 ist, durch Cyclysierung einer Zusammensetzung der Formel (I) in der A $A_3$ ist Y Cl, Br oder J ist und Z H ist, in Gegenwart eines Silbersalzes $Ag_2O$, in dem Fall in dem n = 0 oder 1 ist können die gleichen Verbindungen der Formel (I) in denen A $A_2$ ist auch durch Cyclysierung in basischem Milieu (NaOH, KOH, $K_2CO_3$ und andere) aus Verbindungen der Formel I) hergestellt werden, in denen A $A_3$ ist, Y Cl, Br, J, $OSO_2R_2$ ist, Z gleich H ist,

in dem Fall in dem A ($A_2$) ist und n = 2 oder 1 ist, durch Oxidation von Verbindungen der Formel (I) in denen A ($A_2$) ist und n = 1 oder 0 ist,

m) in dem Fall in dem A $A_2$ ist, n = 2, X = 0 ist, durch Oxidation einer Zusammensetzung der Formel (I) in der A $A_1$ ist, n = 2 ist, durch Oxidationsmittel,
n) in dem Fall in dem A $A_2$ ist und X = 0 ist, durch Dehydratisierung von Verbindungen der Formel (XIII) mit Hilfe von Dehydratisierungsagenzien,
o) in dem Fall in dem A $A_2$ ist oder X S ist, durch Reaktion von Verbindungen der Formel (I) in denen A $A_2$ ist und X O ist mit einem Alkalisalz (im allgemeinen Kalium) eines Thiocyanats.

## Claims

### Claims for the following Contracting States : AT, BE, CH, DE, FR, GB, IT, LI, LU, NL, SE

1. Compounds of formula:

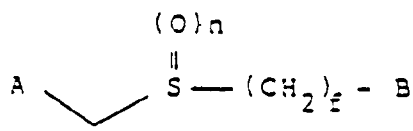

I

in which:
n = 0, 1, 2
f = 0, 1
A is chosen from the groups

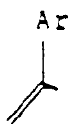

($A_1$)

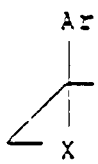

($A_2$)

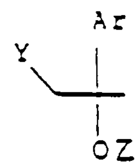

($A_3$)

in which:
Ar is chosen from the groups

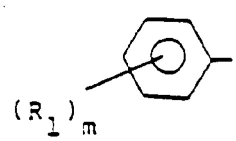

Ar-1

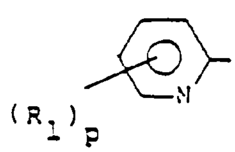

Ar-2

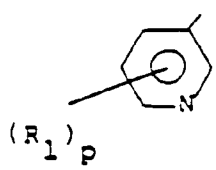

Ar-3

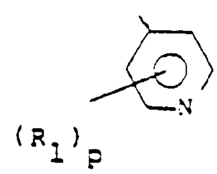

Ar-4

X being an oxygen or sulphur atom,
$R_1$ being a halogen atom (especially Cl or Br or F) or a $C_1$-$C_4$ alkyl, $C_1$-$C_4$ alkoxy, $C_1$-$C_4$ haloalkyl, $C_1$-$C_4$ haloalkoxy, nitro, cyano, $C_6$-$C_{10}$ aryl (especially phenyl or naphthyl), $C_7$-$C_{11}$ aralkyl (especially benzyl), $C_6$-$C_{10}$ aryloxy (especially phenoxy or naphthoxy) optionally substituted by 1 or 2 halogen atoms, or $C_7$-$C_{11}$ aralkyloxy (especially benzyloxy) group, optionally substituted by 1 or 2 halogen atoms,
m = 0, 1, 2, 3, 4, 5
p = 0, 1, 2, 3, 4
the various radicals $R_1$ being identical or different when m or p is greater than or equal to 2.
Y is a chlorine or bromine or iodine atom or an $OSO_2R_2$ group,
$R_2$ being a $C_1$-$C_6$ alkyl, $C_6$-$C_{10}$ aryl or $C_7$-$C_{11}$ aralkyl group, the said groups being optionally substituted by 1 to 3 halogens or $C_1$-$C_4$ alkyl, $C_1$-$C_4$ alkoxy, $C_1$-$C_4$ haloalkyl, $C_1$-$C_4$ haloalkoxy or nitro groups,
Z being a hydrogen atom or a (C=O)$R_{11}$ group,
$R_{11}$ is hydrogen, $C_1$-$C_4$ alkyl, $C_1$-$C_4$ haloalkyl,
B is chosen from the $C_1$-$C_{10}$ alkyl and $C_3$-$C_{10}$ cycloalkyl groups, these groups being optionally substituted

by 1 to 6 halogen atoms or is chosen from the groups

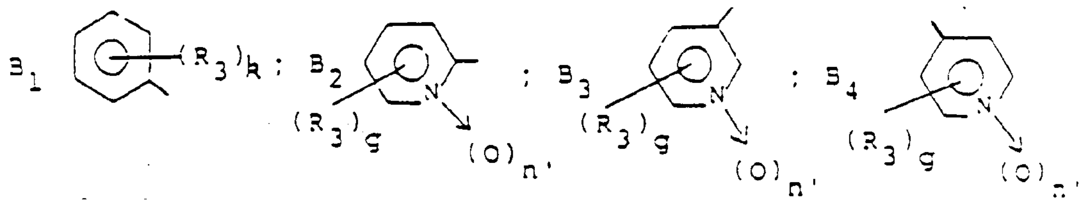

$R_3$ having one of the meanings shown for $R_1$ or $NR_4R_5$, $S(O)_hR_5$ or $(C{=}O)R_7$,
$R_4$ and $R_5$, which are identical or different, are hydrogen, $C_1$-$C_4$ alkyl or $C_6$-$C_{10}$ aryl,
$R_6$ is $C_1$-$C_4$ alkyl,
$R_7$ is $C_1$-$C_4$ alkyl, $C_1$-$C_4$ alkoxy, $C_1$-$C_4$ haloalkoxy or $NR_9R_{10}$,
$R_9$ and $R_{10}$, which are identical or different, are hydrogen or $C_1$-$C_4$ alkyl,
k = 0, 1, 2, 3, 4, 5,
g = 0, 1, 2, 3, 4,
h = 0, 1, 2,
n′= 0, 1.

2. Compound according to Claim 1, characterised in that n = 2.

3. Compound according to Claim 1, characterised in that X = O.

4. Compound according to Claim 1, characterised in that m is smaller than or equal to 2.

5. Compound according to Claim 1, characterised in that p is smaller than or equal to 2.

6. Compound according to Claim 1, characterised in that k is smaller than or equal to 2.

7. Compound according to Claim 1, characterised in that g is smaller than or equal to 1.

8. Compound according to Claim 1, characterised in that $R_1$ is halogen, nitro, trifluoromethyl, methoxy or methyl.

9. Compound according to Claim 1, characterised in that Z is hydrogen.

10. Use of the compounds according to one of Claims 1 to 9 as a herbicide especially in the form of a herbicidal composition additionally comprising an inert carrier.

11. Herbicidal composition comprising 0.05 to 95 % by weight of an active ingredient according to one of Claims 1 to 9 in combination with the agriculturally acceptable solid or liquid carriers and the agriculturally acceptable surface-active agents.

12. Weeding process (especially of dicotyledon crop or maize areas) which consists in applying to the plants which are to be destroyed an effective quantity of a compound of formula (I) according to Claim 1, preferably at pre-emergence.

13. Process for the preparation of the compounds of formula (I) according to Claim 1, characterised in that they are obtained:
a) in the case where n = 2 and A is A1, by bringing a compound of formula:

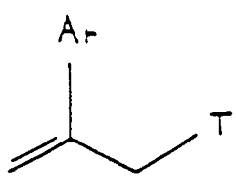

II

in which Ar, $R_1$ and m or p have the same meaning as in Claim 1, and T is a chlorine or bromine atom, into contact with a compound of formula:

$MSO_2(CH_2)_f$-B III

f and B having the same meaning as that shown in Claim 1, M being an alkali metal or alkaline-earth metal atom,

b) in the case where A is A1 and n = 0, 1 or 2 by reaction of the alkali metal salt of an aryl or alkyl thiolate of formula:

M'-S-$(CH_2)_f$B VII

in which M' is an alkali metal or alkaline-earth metal atom, especially Na or R, f and B having the same definition as in Claim 1, with a compound of formula (II) where T is halogen described above in a) in an inert solvent and optional oxidation of the sulphide obtained (n = 0) to a sulphoxide (n = 1) or sulphone (n = 2),

c) in the case where n = 2 and A is A1 by reaction of a compound of formula:

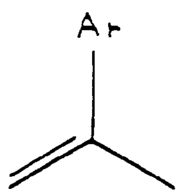

IV

in which Ar is Ar-1 (phenyl nucleus), $R_1$ and m having the same meaning as in Claim 1, with the compound of formula (III) described above in a) with one equivalent of iodine,

d) in the case where A is A1, n = 1 or 2, by bringing the anion of a dialkyl or aryl aryl- or alkylsulphonomethylphosphonate of formula:

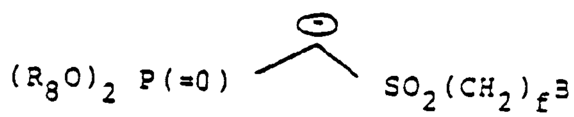

VIII

or the anion of a dialkyl or aryl aryl- or alkylsulphinomethylphosphonate of formula:

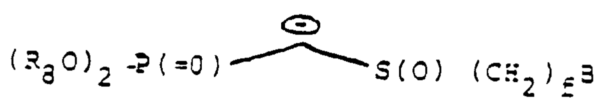

IX

in which formulae $R_8$ is a $C_1$-$C_4$ alkyl or $C_6$-$C_{10}$ aryl group, f and B having the same definition as that shown in Claim 1, into contact with an acetylpyridine or acetophenone of formula:

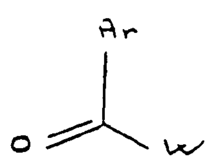

VI

in which Ar, $R_1$ and m or p have the same meaning as in Claim 1 and W is methyl, to yield the compounds of formula X:

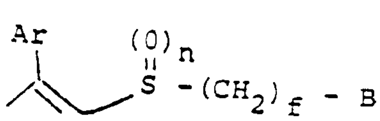

X

in which Ar, n, f and B have the same meaning as in Claim 1, the said compound of formula X then being subjected to an isomerisation stage with 2 or more equivalents of base,

e) in the case where A is A1 and n = 2, in a first stage; by bringing the anion or dianion of a methylaryl

or alkyl sulphone of formula:

$$CH_3SO_2\,(CH_2)_f\text{-}B \qquad \text{IIIB}$$

f and B having the same definition as that shown in Claim 1, into contact with an acetophenone or an acetylpyridine of formula (VI) in which W is methyl and Ar, $R_1$ and m or p have the same definitions as in Claim 1, to yield the Compounds of formula:

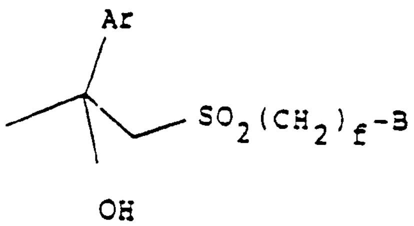

XI

in which Ar, $R_1$, m or p, f and B have the same meaning as in Claim 1,
then in a second stage by dehydration of the compound of formula XI,
f) in the case where A is $A_3$, Y is Cl or Br, and Z is H, by halogenation in aqueous medium of the compounds of formula (I) where A is $A_1$
g) in the case where A is $A_3$, Y is Cl, Br or I, and Z is H, by nucleophilic substitution of the compounds of formula (I) in which A is $A_3$, Y is $OSO_2R_2$, Cl or Br, and Z is H, using the alkali metal (sodium, potassium, lithium) halide salt (Y = Cl, Br, I)
h) in the case where A is $A_3$, Y is Cl or Br, Z is H and n = 2, by bringing the anion of a methylaryl or alkyl sulphone of formula (IIIB), f and B having the same definition as that shown in Claim 1, into contact with an ω-haloacetophenone or haloacetylpyridine of formula:

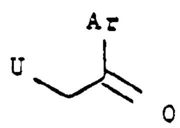

XII

in which U is a chlorine or bromine atom, Ar, $R_1$, m or p having the same definition as that shown in Claim 1,
i) in the case where A is $A_3$, Y is Br or Cl, and Z is H, by opening of the compounds of formula (I) in which A is $A_2$, X is O, by the action of a reactant which gives a halide Y, Y being the chlorine or bromine atom,
j) in the case where A is $A_3$, Y is Br or Cl, and Z is ester, by halogenation, using the N-halosuccinimide or N-haloacetamide, of the compounds of formula (I) where A is $A_1$, previously obtained, in the presence of an acid $R_{11}CO_2H$, $R_{11}$ having the same definition as that shown in Claim 1
in the case where A is $A_3$, Y is I, and Z is ester, by nucleophilic substitution of the compounds of formula (I) where A is $A_3$, Y is Br, and Z is ester, according to the method described in method g.
k) in the case where A is $A_3$, Y is $OSO_2R_2$, and Z is H, by reaction of the halide $R_2SO_2$-Hal (Hal being preferably the chlorine atom) with the diol compound of formula:

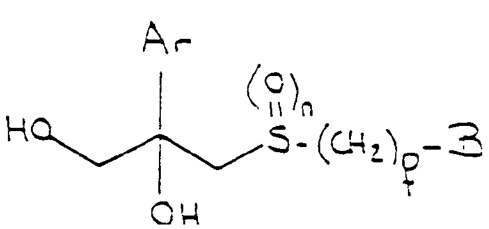

XIII

in the presence of a nitrogenous base such as pyridine or triethylamine,
l) in the case where A is $A_2$, X is O, by ring closure of a compound of formula (I) where A is $A_3$ and Y is Cl, Br or I, and Z is H, in the presence of a silver salt $Ag_2O$, in the case where n = 0 or 1, these same compounds of formula (I) in which A is $A_2$ can also be prepared by ring closure in a basic medium (NaOH, KOH, $K_2CO_3$ and others) of the compounds of formula (I) where A is $A_3$, Y is Cl, Br, I or $OSO_2R_2$, and

Z is H,

in the case where A is ($A_2$) and n = 2 or 1, by oxidation of the compounds of formula (I) where A is ($A_2$) and n = 1 or 0,

m) in the case where A is $A_2$, n = 2, and X = O, by oxidation of a compound of formula (I) where A is $A_1$, n = 2, using oxidising agents,

n) in the case where A is $A_2$ and X = O, by dehydration of the compounds of formula (XIII), using dehydrating agents,

o) in the case where A is $A_2$ and X is S, by reaction of the compounds of formula (I) where A is $A_2$ and X is O with an alkali metal (generally potassium) thiocyanate salt.

**14.** Compounds of formulae X, XI and XIII according to Claim 13, which can be used particularly for implementing the process according to Claim 13.

**Claims for the following Contracting States : ES, GR**

**1.** Herbicidal composition comprising a compound of formula:

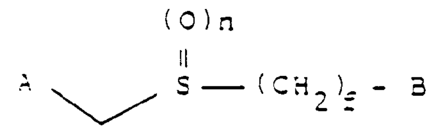

I

in which:
n = 0, 1, 2
f = 0, 1
A is chosen from the groups

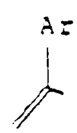
($A_1$)

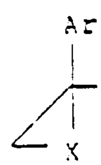
($A_2$)

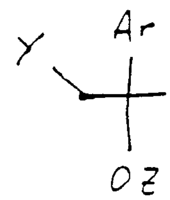
(A3)

in which:
Ar is chosen from the groups

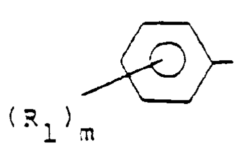
Ar-1

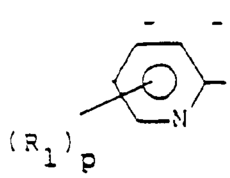
Ar-2

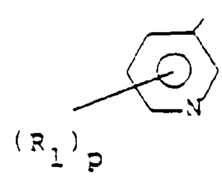
Ar-3

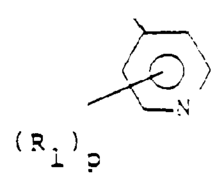
Ar-4

X being an oxygen or sulphur atom,

$R_1$ being a halogen atom (especially Cl or Br or F) or a $C_1$-$C_4$ alkyl, $C_1$-$C_4$ alkoxy, $C_1$-$C_4$ haloalkyl, $C_1$-$C_4$ haloalkoxy, nitro, cyano, $C_6$-$C_{10}$ aryl (especially phenyl or naphthyl), $C_7$-$C_{11}$ aralkyl (especially benzyl), $C_6$-$C_{10}$ aryloxy (especially phenoxy or naphthoxy) optionally substituted by 1 or 2 halogen atoms, or $C_7$-$C_{11}$ aralkyloxy (especially benzyloxy) group, optionally substituted by 1 or 2 halogen atoms,

m = 0, 1, 2, 3, 4, 5

p = 0, 1, 2, 3, 4

the various radicals $R_1$ being identical or different when m or p is greater than or equal to 2.

Y is a chlorine or bromine or iodine atom or an $OSO_2R_2$ group,

$R_2$ being a $C_1$-$C_6$ alkyl, $C_6$-$C_{10}$ aryl or $C_7$-$C_{11}$ aralkyl group, the said groups being optionally substituted by 1 to 3 halogens or $C_1$-$C_4$ alkyl, $C_1$-$C_4$ alkoxy, $C_1$-$C_4$ haloalkyl, $C_1$-$C_4$ haloalkoxy or nitro groups,

Z being a hydrogen atom or a (C=O)$R_{11}$ group,
$R_{11}$ is hydrogen, $C_1$-$C_4$ alkyl, $C_1$-$C_4$ haloalkyl,
B is chosen from the $C_1$-$C_{10}$ alkyl and $C_1$-$C_{10}$ cycloalkyl groups, these groups being optionally substituted by 1 to 6 halogen atoms or is chosen from the groups

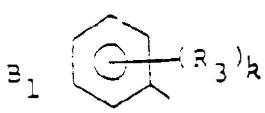 ; 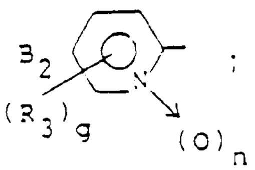 ; 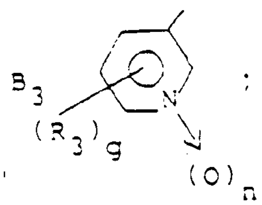 ; 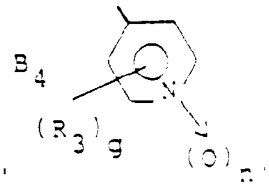

$R_3$ having one of the meanings shown for $R_1$ or $NR_4R_5$,
$S(O)_hR_6$ or $(C=O)R_7$,
$R_4$ and $R_5$, which are identical or different, are hydrogen, $C_1$-$C_4$ alkyl or $C_6$-$C_{10}$ aryl,
$R_6$ is $C_1$-$C_4$ alkyl,
$R_7$ is $C_1$-$C_4$ alkyl, $C_1$-$C_4$ alkoxy, $C_1$-$C_4$ haloalkoxy or $NR_9R_{10}$,
$R_8$ and $R_{10}$, which are identical or different, are hydrogen or $C_1$-$C_4$ alkyl,
k = 0, 1, 2, 3, 4, 5,
g = 0, 1, 2, 3, 4,
h = 0, 1, 2,
n' = 0, 1.
with an inert carrier.

2. Composition according to Claim 1, characterised in that n = 2.

3. Composition according to Claim 1, characterised in that X = O.

4. Composition according to Claim 1, characterised in that m is smaller than or equal to 2.

5. Composition according to Claim 1, characterised in that p is smaller than or equal to 2.

6. Composition according to Claim 1, characterised in that k is smaller than or equal to 2.

7. Composition according to Claim 1, characterised in that g is smaller than or equal to 1.

8. Composition according to Claim 1, characterised in that $R_1$ is halogen, nitro, trifluoromethyl, methoxy or methyl.

9. Composition according to Claim 1, characterised in that Z is hydrogen.

10. Use of the compounds according to one of Claims 1 to 9 as a herbicide.

11. Herbicidal composition comprising 0.05 to 95 % by weight of an active ingredient according to one of Claims 1 to 9 in combination with the agriculturally acceptable solid or liquid carriers and the agriculturally acceptable surface-active agents.

12. Weeding process (especially of dicotyledon crop or maize areas) which consists in applying to the plants which are to be destroyed an effective quantity of a compound of formula (I) according to Claim 1, preferably at pre-emergence.

13. Process for the preparation of the compounds of formula (I) according to Claim 1, characterised in that they are obtained:
a) in the case where n = 2 and A is A1, by bringing a compound of formula:

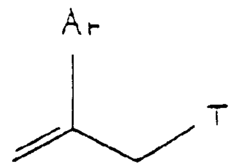

II

in which Ar, $R_1$ and m or p have the same meaning as in Claim 1, and T is a chlorine or bromine atom, into contact with a compound of formula:

$MSO_2(CH_2)_f$-B III

f and B having the same meaning as that shown in Claim 1, M being an alkali metal or alkaline-earth metal atom,

b) in the case where A is A1 and n = 0, 1 or 2 by reaction of the alkali metal salt of an aryl or alkyl thiolate of formula:

$M'$-S-$(CH_2)_f$B VII

in which M' is an alkali metal or alkaline-earth metal atom, especially Na or K, f and B having the same definition as in Claim 1, with a compound of formula (II) where T is halogen described above in a) in an inert solvent and optional oxidation of the sulphide obtained (n = 0) to a sulphoxide (n = 1) or sulphone (n = 2),

c) in the case where n = 2 and A is A1 by reaction of a compound of formula:

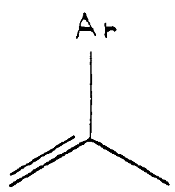

IV

in which Ar is Ar-1 (phenyl nucleus), $R_1$ and m having the same meaning as in Claim 1, with the compound of formula (III) described above in a) with one equivalent of iodine,

d) in the case where A is A1, n = 1 or 2, by bringing the anion of a dialkyl or aryl aryl- or alkylsulphonomethylphosphonate of formula:

$(R_8O)_2$ P(=O) $SO_2(CH_2)_f$B VIII

or the anion of a dialkyl or aryl aryl- or alkylsulphinomethylphosphonate of formula:

$(R_8O)_2$ P(=O) S(O) $(CH_2)_f$B IX

in which formulae $R_8$ is a $C_1$-$C_4$ alkyl or $C_6$-$C_{10}$ aryl group, f and B having the same definition as that shown in Claim 1, into contact with an acetylpyridine or acetophenone of formula:

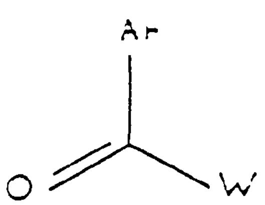

VI

in which Ar, $R_1$ and m or p have the same meaning as in Claim 1 and W is methyl, and isomerisation of the vinyl compound obtained with 2 or more equivalents of base,

e) in the case where A is A1 and n = 2, in a first stage; by bringing the anion or dianion of a methylaryl or alkyl sulphone of formula:

$CH_3SO_2(CH_2)_f$-B IIIB

f and B having the same definition as that shown in Claim 1, into contact with an acetophenone or an acetylpyridine of formula (VI) in which W is methyl and Ar, $R_1$ and m or p have the same definitions as in Claim 1, to yield the compounds of formula:

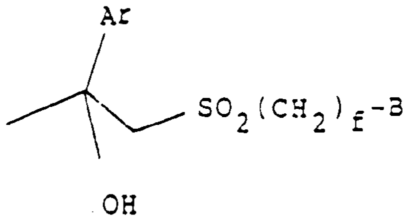

XI

in which Ar, $R_1$, m or p, f and B have the same meaning as in Claim 1, then in a second stage by dehydration of the compound of formula XI,

f) in the case where A is $A_3$, Y is Cl or Br, and Z is H, by halogenation in aqueous medium of the compounds of formula (I) where A is $A_1$

g) in the case where A is $A_3$, Y is Cl, Br or I, and Z is H, by nucleophilic substitution of the compounds of formula (I) in which A is $A_3$, Y is $OSO_2R_2$, Cl or Br, and Z is H, using the alkali metal (sodium, potassium, lithium) halide salt (Y = Cl, Br, I)

h) in the case where A is $A_3$, Y is Cl or Br, Z is H and n = 2, by bringing the anion of a methylaryl or alkyl sulphone of formula (IIIB), f and B having the same definition as that shown in Claim 1, into contact with an ω-haloacetophenone or haloacetylpyridine of formula:

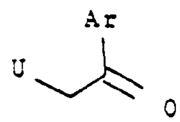

XII

in which U is a chlorine or bromine atom, Ar, $R_1$, m or p having the same definition as that shown in Claim 1,

i) in the case where A is $A_3$, Y is Br or Cl, and Z is H, by opening of the compounds of formula (I) in which A is $A_2$, X is O, by the action of a reactant which gives a halide Y, Y being the chlorine or bromine atom,

j) in the case where A is $A_3$, Y is Br or Cl, and Z is ester, by halogenation, using the N-halosuccinimide or N-haloacetamide, of the compounds of formula (I) where A is $A_1$, previously obtained, in the presence of an acid $R_{11}CO_2H$, $R_{11}$ having the same definition as that shown in Claim 1

in the case where A is $A_3$, Y is I, and Z is ester, by nucleophilic substitution of the compounds of formula (I) where A is $A_3$, Y is Br, and Z is ester, according to the method described in method g.

k) in the case where A is $A_3$, Y is $OSO_2R_2$, and Z is H, by reaction of the halide $R_2SO_2$-Hal (Hal being preferably the chlorine atom) with the diol compound of formula:

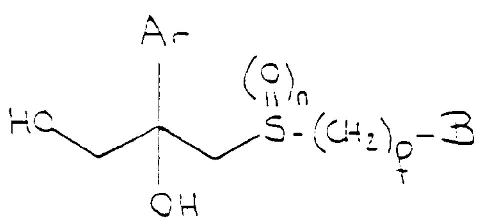

XIII

in the presence of a nitrogenous base such as pyridine or triethylamine,

l) in the case where A is $A_2$, X is O, by ring closure of a compound of formula (I) where A is $A_3$ and Y is Cl, Br or I, and Z is H, in the presence of a silver salt $Ag_2O$, in the case where n = 0 or 1, these same compounds of formula (I) in which A is $A_2$ can also be prepared by ring closure in a basic medium (NaOH, KOH, $H_2CO_3$ and others) of the compounds of formula (I) where A is $A_3$, Y is Cl, Br, I or $OSO_2R_2$, and Z is H,

in the case where A is ($A_2$) and n = 2 or 1, by oxidation of the compounds of formula (I) where A is ($A_2$) and n = 1 or 0,

m) in the case where A is $A_2$, n = 2, and X = O, by oxidation of a compound of formula (I) where A is $A_1$, n = 2, using oxidising agents,

n) in the case where A is $A_2$ and X = O, by dehydration of the compounds of formula (XIII), using dehydrating agents,

o) in the case where A is $A_2$ and X is S, by reaction of the compounds of formula (I) where A is $A_2$ and X is O with an alkali metal (generally potassium) thiocyanate salt